(19) Europäisches Patentamt
European Patent Office
Office européen des brevets

(11) EP 0 942 994 B1

(12) EUROPEAN PATENT SPECIFICATION

(45) Date of publication and mention of the grant of the patent:
**20.04.2005 Bulletin 2005/16**

(21) Application number: **96945189.7**

(22) Date of filing: **09.12.1996**

(51) Int Cl.$^7$: **C12N 15/56**, C12N 9/28, C11D 3/386, A23K 1/00

(86) International application number:
**PCT/US1996/019595**

(87) International publication number:
**WO 1998/026078 (18.06.1998 Gazette 1998/24)**

(54) **H MUTANT ALPHA-AMYLASE ENZYMES**

MUTIERTE ALPHA-AMYLASE ENZYME MIT ERHÖHTER STABILITÄT

ENZYMES ALPHA-AMYLASE H-MUTANTES

(84) Designated Contracting States:
**AT BE CH DE DK ES FI FR GB GR IE IT LI LU MC NL PT SE**

(43) Date of publication of application:
**22.09.1999 Bulletin 1999/38**

(73) Proprietor: **GENENCOR INTERNATIONAL, INC.**
**Palo Alto, California 94304 (US)**

(72) Inventors:
- **DAY, Anthony G., Genencor International, Inc. Palo Alto, CA 94304-1013 (US)**
- **MITCHINSON, Colin, Genencor International, Inc. Palo Alto, CA 94304-1013 (US)**
- **SHAW, Andrew, Genencor International, Inc. Palo Alto, CA 94304-1013 (US)**

(74) Representative: **Kremer, Simon Mark et al**
**Mewburn Ellis LLP**
**York House,**
**23 Kingsway**
**London WC2B 6HP (GB)**

(56) References cited:
**WO-A-95/10603**
**WO-A-95/35382**
**WO-A-96/23874**
**WO-A-96/39528**

- **BIOMED BIOCHIM ACTA, 50 (2). 1991. 213-218., XP002039104 MAASSEN A: "COMPARISON OF TWO ALPHA AMYLASES FROM BACILLUS-AMYLOLIQUEFACIENS AND BACILLUS-LICHENIFORMIS"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 942 994 B1

## Description

### FIELD OF THE INVENTION

The present invention is directed to improved proteins, and particularly enzymes, having altered stability characteristics through the recruitment of residues from a less stable homolog or related protein. The present invention is particularly directed to novel mutant α-amylase enzymes derived from *B. licheniformis* having at least one recruitment from a corresponding residue in *B. stearothermophilus* and/or *B. amyloliquefaciens* which differs from the residue in *B. licheniformis*. The resultant α-amylases exhibit improved stability under adverse conditions.

### BACKGROUND OF THE INVENTION

Methods of improving the stability of proteins have been utilized extensively for the purpose of increasing the usefulness of the proteins in practice. Such techniques often involve the use of protein engineering and site-specific mutagenesis to isolate and select specific amino acid residues which appear, based on the specific amino acid properties and/or the structure of the protein, to be especially relevant or are shown to be relevant through experimental evidence. For example, Estell et al., U.S. Patent No. 4,760,025 suggest modification of oxidatively unstable enzymes to confer increased stability by altering residues which are particularly susceptible to oxidation, i.e., methionine, lysine, tryptophan and cysteine, among others. Koths et al., U.S. Patent No. 4,752,585, suggest a method of improving the oxidation stability of a therapeutic protein by making a conservative amino acid substitution for each methionyl residue susceptible to chloramine T oxidation. Barr et al., U.S. Patent No. 4,732,973, suggest substituting the active site methionine from human $\alpha_1$-antitrypsin with an oxidatively stable amino acid.

Additional techniques which have been suggested include homology modeling of proteins against evolutionarily close enzymes to provide a basis for protein engineering. Siezen et al., Protein Engineering, vol. 4, no. 7, pp. 719-737 (1991) disclose a strategy for improving the properties of proteases used in industrial processes by incorporating or duplicating features from more stable enzymes into a target.

Nonetheless, protein engineering based on homology modeling requires a relatively high degree of homology (greater than 70%) to be considered highly successful. It has further been reported that, where the homology falls to below 30%, such modeling becomes far less successful (see e.g., Machius et al., J. Mol. Biol., vol. 246, p. 547 (1995)).

Of particular interest to industry, are the α-amylases (α-1,4-glucan-4-glucanohydrolase, EC 3.2.1.1) for which increased stability and/or activity are desired. α-Amylases hydrolyze internal α-1,4-glucosidic linkages in starch, largely at random, to produce smaller molecular weight malto-dextrins. α-Amylases are of considerable commercial value, being used in the initial stages (liquefaction) of starch processing; in alcohol production; as cleaning agents in detergent matrices; and in the textile industry for starch desizing. α-Amylases are produced by a wide variety of bacterial, fungal and plant sources including *Bacillus* and *Aspergillus*, with most commercial amylases used in the starch processing industry being produced from bacterial sources such as *Bacillus licheniformis, Bacillus amyloliquefaciens, Bacillus subtilis,* or *Bacillus stearothermophilus*. In recent years, the preferred enzymes in commercial use have been those from *Bacillus licheniformis* because of their heat stability and performance, at least at neutral and mildly alkaline pH's. Amylases derived from related species such as *Bacillus amyloliquefaciens, Bacillus subtilis* and *Bacillus stearothermophilus* are generally believed to be considerably less stable under many conditions.

Variant α-amylases have been suggested in PCT Publication No. WO 95/10603, which have improved laundry or dishwashing performance and comprise a mutation other than a single mutation at position M197 in *Bacillus licheniformis* α-amylase. In PCT Publication No. WO 94/02597, a mutant α-amylase having improved oxidative stability is described wherein one or more methionines are replaced by any amino acid except cysteine or methionine. In PCT publication No. WO 94/18314, a mutant α-amylase having improved oxidative stability is described wherein one or more of the methionine, tryptophan, cysteine, histidine or tyrosine residues is replaced with a non-oxidizable amino acid. In PCT Publication No. WO 91/00353, the performance characteristics and problems associated with starch liquefaction with wild type *Bacillus licheniformis* α-amylase are approached by genetically engineering the α-amylase to include the specific substitutions Ala-111-Thr, His-133-Tyr and/or Thr-149-Ile.

Studies using recombinant DNA techniques to explore which residues are important for the catalytic activity of amylases and/or to explore the effect of modifying certain amino acids within the active site of various amylases and glycosylases have been conducted by various researchers (Vihinen et al., J. Biochem., vol. 107, pp. 267-272 (1990); Holm et al., Protein Engineering, vol. 3, pp. 181-191 (1990); Takase et al., Biochemica et Biophysica Acta, vol. 1120, pp. 281-288 (1992); Matsui et al., Febs Letters, vol. 310. pp. 216-218 (1992); Matsui et al., Biochemistry, vol. 33, pp. 451-458 (1992); Sogaard et al., J. Biol. Chem., vol. 268, pp. 22480-22484 (1993); Sogaard et al., Carbohydrate Polymers, vol. 21, pp. 137-146 (1993); Svensson, Plant Mol. Biol., vol. 25, pp. 141-157 (1994); Svensson et al., J. Biotech. vol. 29, pp. 1-37 (1993)). Researchers have also studied which residues are important for thermal stability (Suzuki et al., J. Biol. Chem. vol. 264, pp. 18933-18938 (1989); Watanabe et al., Eur. J. Biochem. vol. 226, pp. 277-283 (1994));

and one group has used such methods to introduce mutations at various histidine residues in a *Bacillus licheniformis* amylase, the rationale being that *Bacillus licheniformis* amylase which is known to be relatively thermostable when compared to other similar *Bacillus* amylases, has an excess of histidines and, therefore, it was suggested that replacing a histidine could affect the thermostability of the enzyme. This work resulted in the identification of stabilizing mutations at the histidine residue at the +133 position and the alanine residue at position +209 (Declerck et al., J. Biol. Chem., vol. 265, pp. 15481-15488 (1990); FR 2 665 178-A1; Joyet et al., Bio/Technology, vol. 10, pp. 1579-1583 (1992)).

Thus, there has been a considerable effort in the field of protein engineering to produce more stable enzymes. Such techniques, including recruitment from more stable enzymes to less stable enzymes, random mutagenesis and sequence alignment and homology modeling have thus far produced many successes. However, there remains a need in the field for additional techniques useful for the production of improved enzymes.

## SUMMARY OF THE INVENTION

It is an object of the present invention to provide an α-amylase which has increased stability.

It is a further object of the present invention to provide an α-amylase, having altered stability profiles, such as pH stability, alkaline stability, oxidative stability and/or thermostability.

According to the present invention, an improved α-amylase is provided comprising an amino acid sequence which has been altered from a precursor amino acid sequence by the substitution of an amino acid residue which differs from a corresponding amino acid residue in a less stable but homologous protein. The improved α-amylase has improved stability properties compared to a protein corresponding to the precursor amino acid sequence. Preferably, the substitution does not occur at a buried residue but instead at a residue on the surface of the molecule.

In the invention, the α-amylase is derived from *Bacillus licheniformis* and the less stable but homologous protein comprises either or both of the amylases derived from *Bacillus amyloliquefaciens* or *Bacillus stearothermophilus*.

In the invention, the precursor protein is α-amylase derived from *Bacillus licheniformis* and the substituted residues in the improved α-amylase, which differ in both *Bacillus stearothermophilus* and *Bacillus amyloliquefaciens*. comprise one or more of the following specific substitutions which occur naturally in both *Bacillus stearothermophilus* and *Bacillus amyloliquefaciens*, i.e., A33 S, A52 S, N96 Q, H133 Y, S148 N, A209 V, A269 K, A379 S and/or A435 S.

An advantage of the present invention is that it is possible to produce more stable protein through a simple technique of analyzing the aligned sequences of two homologous but stability differentiated proteins.

## BRIEF DESCRIPTION OF THE DRAWINGS

Figure 1 illustrates mutagenic oligonucleotides useful during directed mutagenesis of Asn188 from *Bacillus licheniformis* α-amylase. In this and following figures illustrating oligonucleotide constructs, bold letters indicate base changes introduced by the oligonucleotide and underlining indicates restriction endonuclease sites introduced by the oligonucleotide.

Figure 2 illustrates PCR primers used for PCR processing of mutagenic oligonucleotide templates.

Figure 3 illustrates the DNA sequence of the gene for α-amylase from *Bacillus licheniformis* (NCIB 8061) (SEQ ID NO:33) and deduced amino acid sequence of the translation product (SEQ ID NO:41) as described by Gray et al., J. Bacteriology, vol. 166, pp. 635-643 (1986).

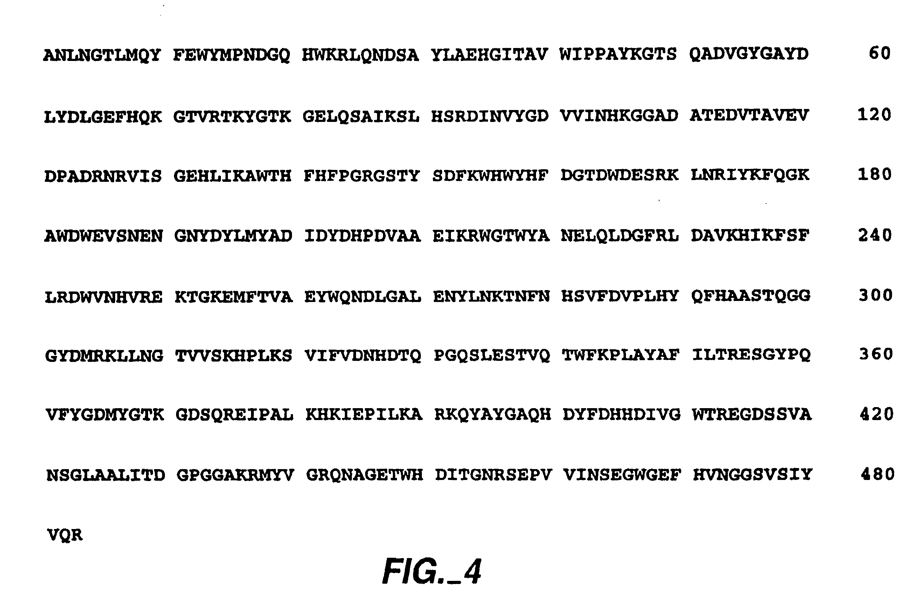

Figure 4 illustrates the amino acid sequence (SEQ ID NO:34) of the mature α-amylase enzyme from *Bacillus licheniformis*.

Figure 5 illustrates an alignment of the primary structures of three *Bacillus* α-amylases. The *Bacillus licheniformis* α-amylase (Am-Lich) (SEQ ID NO:35) is described by Gray et al., J. Bacteriology, vol. 166, pp. 635-643 (1986); the *Bacillus amyloliquefaciens* α-amylase (Am-Amylo) (SEQ ID NO:36) is described by Takkinen et al., J. Biol. Chem., vol. 258, pp. 1007-1013 (1983); and the *Bacillus stearothermophilus* α-amylase (Am-Stearo) (SEQ ID NO: 37) is described by Ihara et al., J. Biochem., vol. 98, pp. 95-103 (1985).

Figure 6 illustrates plasmid pHP13 wherein $Cm^R$ refers to chloramphenicol resistance, $Em^R$ refers to erythromycin resistance and Rep pTA1060 refers to the origin of replication from plasmid pTA1060.

Figure 7 illustrates the pBLapr plasmid wherein BL AA refers to *Bacillus licheniformis* α-amylase gene; *aprE* refers to the promoter and signal peptide encoding region of the *aprE* gene; AmpR refers to the ampicillin resistant gene from pBR322; and CAT refers to the chloramphenicol resistance gene from pC194.

Figure 8 illustrates the pHP.BL plasmid carrying the gene for *Bacillus licheniformis* α-amylase.

Figure 9 illustrates a schematic of the PCR method used to produce the mutant oligonucleotides corresponding to α-amylase derived from *Bacillus licheniformis*.

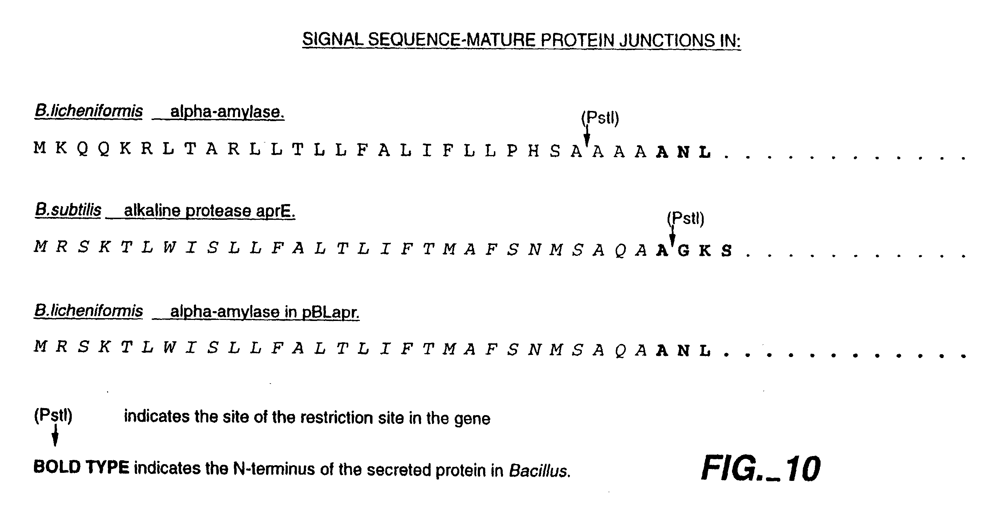

Figure 10 illustrates the signal sequence-mature protein junctions in α-amylase derived from *Bacillus licheniformis* (SEQ ID NO:38), *Bacillus subtilis aprE* (SEQ ID NO: 39) and *Bacillus licheniformis* in pBLapr (SEQ ID NO:40).

DETAILED DESCRIPTION

"Expression vector" means a DNA construct comprising a DNA sequence which is operably linked to a suitable control sequence capable of effecting the expression of said DNA in a suitable host. Such control sequences may include a promoter to effect transcription, an optional operator sequence to control such transcription, a sequence encoding suitable mRNA ribosome-binding sites, and sequences which control termination of transcription and translation. Where expression is desired in a *Bacillus* host, a preferred promoter is the *Bacillus subtilis aprE* promoter. The vector may be a plasmid, a phage particle, or simply a potential genomic insert. Once transformed into a suitable host, the vector may replicate and function independently of the host genome, or may, in some instances, integrate into the genome itself. While plasmid and vector are sometimes used interchangeably as the plasmid is the most commonly used form of vector at present, the invention is intended to include such other forms of expression vectors which serve equivalent functions and which are, or become, known in the art.

"Host strain" or "host cell" means a suitable host for an expression vector comprising DNA encoding a protein according to the present invention. Host cells useful in the present invention are generally procaryotic or eucaryotic hosts, including any transformable microorganism in which the expression of a given protein according to the present invention can be achieved. One of skill in the art will be familiar with the appropriate expression and secretion machinery, including the appropriate host cell, for use with a specific protein. For example, host strains of the same species or genus from which the particular protein is derived are suitable, for example with α-amylase derived from *Bacillus*, a suitable host cell would be a *Bacillus* strain. In this case, an α-amylase negative *Bacillus* strain (genes deleted) and/or an α-amylase and protease deleted *Bacillus* strain (Δ*amyE,* Δ*apr,* Δ*npr*) is preferably used. Host cells are transformed or transfected with vectors constructed using recombinant DNA techniques. Such transformed host cells are capable of either replicating vectors encoding the protein and its variants (mutants) or expressing the desired protein.

"Recombinant protein" means a protein in which the DNA sequence encoding the naturally occurring or precursor protein is modified to produce a mutant DNA sequence which encodes the substitution, insertion or deletion of one or more amino acids in the protein sequence compared to the naturally occurring or precursor protein. As used herein, the term precursor protein (or enzyme) refers not to an immediate precursor in terms of a chemical reaction, but instead to a parent protein from which the modification is modeled.
Accordingly, while it is the precursor which defines the modification, the actual alteration or modification will have its basis in an alteration within the DNA which encodes the precursor, which DNA is then transformed, expressed and the protein product secreted which incorporates the modification.

The improved α-amylase according to the present invention comprises an amino acid sequence which is derived from the amino acid sequence of a precursor protein. The precursor protein may be a naturally occurring protein or a recombinant protein. The amino acid sequence of the improved α-amylase is derived from the precursor protein's amino acid sequence by the substitution of one or more amino acids of the precursor amino acid sequence. Such modification is generally of the precursor DNA sequence which encodes the amino acid sequence of the precursor proteins rather than manipulation of the precursor protein *per se.* Suitable methods for such manipulation of the precursor DNA sequence include methods disclosed herein and in commonly owned U.S. Patent Nos. 4,760,025 and 5,185,258, incorporated herein by reference.

A protein may be modified as follows. The protein is referenced to a homologous protein for differences in sequence. For example, it is possible to line up the sequences of two related proteins according to well known sequence alignment techniques and determine conserved (i.e., identical) positions as well as the positions at which the two proteins differ. It has surprisingly and unexpectedly been found that by referencing the target protein against a less stable protein and identifying and selecting certain residues which differ from the less stable protein for modification in the target protein, advantageous improvements in stability may be obtained. It is necessary that the two proteins have comparable activity or function and be substantially homologous. By comparable activity, the proteins should have similar biological activity, function, catalytic activity or such other criteria as are commonly used to classify a specific protein. "Substantially homologous" means that the proteins have a significant level of conserved, i.e., identical, amino acids such that their sequences can be meaningfully aligned and major structural, functional or catalytic sites defined. Preferably, the two proteins have a sequence identity of at least 60%, more preferably 65% sequence identity, and most preferably 80% sequence identity.

The improved α-amylase of the invention should have stability which is at least 50% of the precursor protein, more preferably at least 70%, and most preferably at least 90% of the precursor protein under any given set of circumstances, yet having improved stability under other conditions. Thus, an improved α-amylase which has a stability of at least 50% of the precursor enzyme under high temperature but which has improved stability under oxidative conditions would be within the scope of the invention. In a particularly preferred embodiment, the stability of the improved α-amylase is improved with respect to the precursor protein in the presence of oxidants and high temperature. "α-Amylase" as used herein means an enzymatic activity which cleaves or hydrolyzes the α(1-4)glycosidic bond, e.g., that in starch, amylopectin or amylose polymers. α-Amylase includes naturally occurring α-amylases as well as recombinant α-amylases. α-amylases in the present invention are those derived from *Bacillus licheniformis*. Accordingly, the α-amylases according to the present invention are derived from a precursor α-amylase. The precursor α-amylase is produced by any source capable of producing α-amylase. Suitable sources of α-amylases are prokaryotic or eukaryotic organisms, including fungi, bacteria, plants or animals. The precursor α-amylase is produced by *Bacillus licheniformis*.

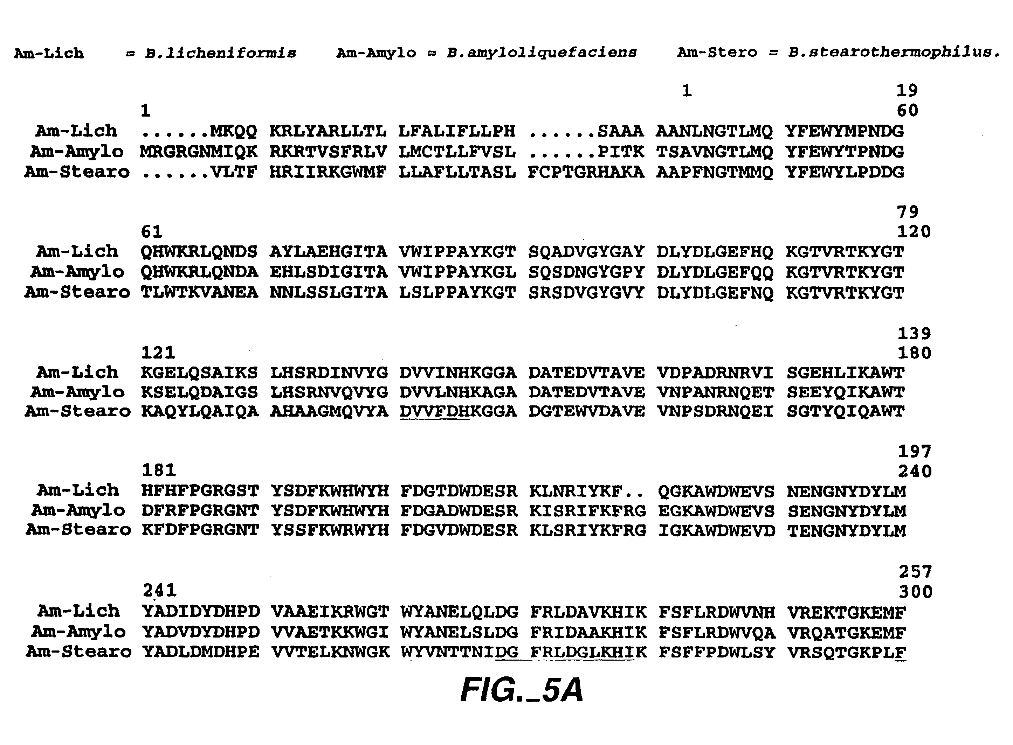

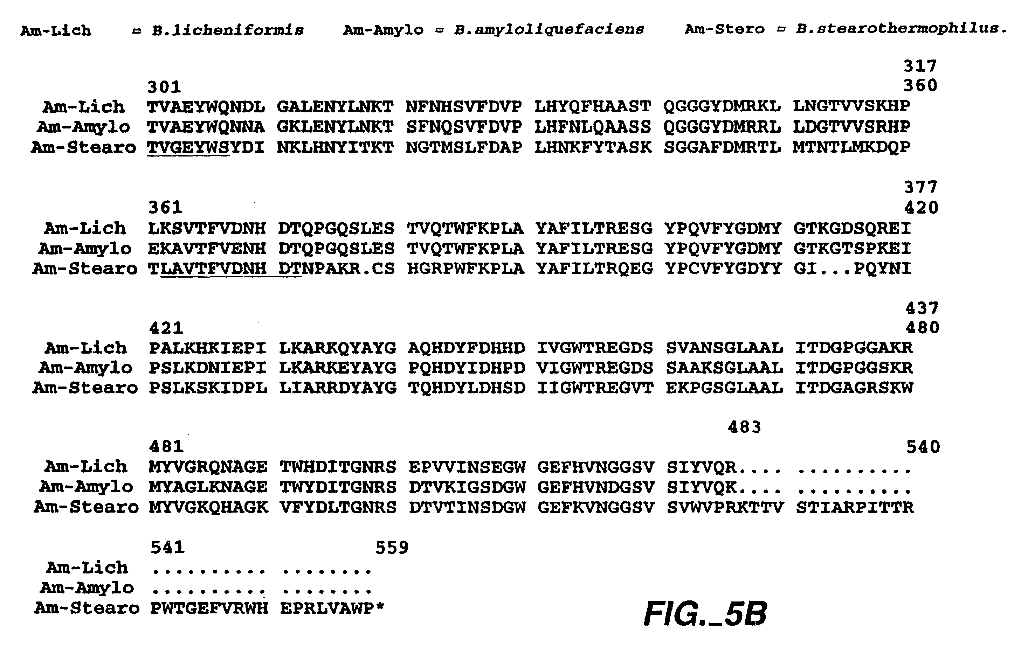

Homologies have been found between almost all endo-amylases sequenced to date, ranging from plants, mammals, and bacteria (Nakajima et al., Appl. Microbiol. Biotechnol., vol. 23, pp. 355-360 (1986); Rogers, Biochem. Biophys. Res. Commun., vol. 128, pp. 470-476 (1985); Janecek, Eur. J. Biochem., vol. 224, pp. 519-524 (1994)). There are four areas of particularly high homology in certain *Bacillus* amylases, as shown in Figure 5, wherein the underlined sections designate the areas of high homology. Sequence alignments have also been used to map the relationship between *Bacillus* endo-amylases (Feng et al., J. Molec. Evol., vol. 35, pp. 351-360 (1987)). The relative sequence homology between *Bacillus stearothermophilus* and *Bacillus licheniformis* amylase is about 66% and that between *Bacillus licheniformis* and *Bacillus amyloliquefaciens* amylases is about 81%, as determined by Holm et al., Protein Engineering, vol. 3, No. 3, pp. 181-191 (1990).

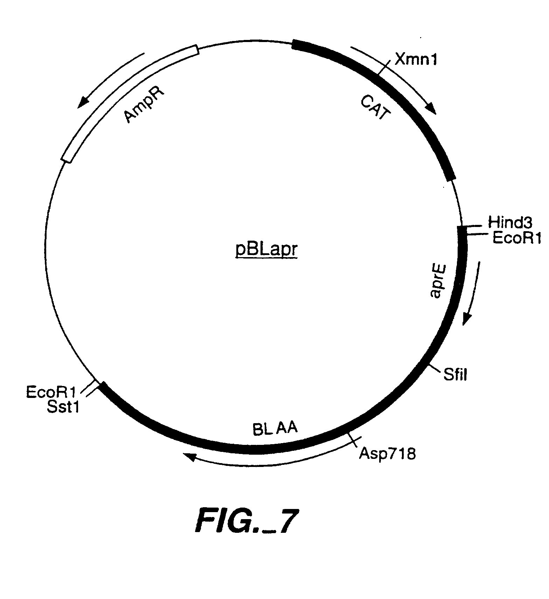

In order to establish homology to primary structure, the amino acid sequence of a precursor α-amytase is directly compared to the *Bacillus licheniformis* α-amylase primary sequence and particularly to a set of residues known to be invariant to all α-amylases for which sequences are known (see e.g., Figure 7). It is possible also to determine equivalent residues by tertiary structure analysis of the crystal structures reported for porcine pancreatic α-amylase (Buisson et al., EMBO Journal, vol. 6, pp. 3909-3916 (1987); Qian et al., Biochemistry, vol. 33, pp. 6284-6294 (1994); Larson et al., J. Mol. Biol., vol. 235, pp. 1560-1584 (1994)); Taka-amylase A from *Aspergillus oryzae* (Matsuura et al., J. Biochem. (Tokyo), vol. 95, pp. 697-702 (1984)); and an acid α-amylase from *A. niger (Boel* et al., Biochemistry, vol. 29, pp. 6244-6249 (1990)), with the former two structures being similar, and for barley α-amylase (Vallee et al., J. Mol. Biol., vol. 236, pp. 368-371(1994); Kadziola, J. Mol. Biol., vol. 239, pp. 104-121 (1994)). Although there have been some preliminary studies published (Suzuki et al, J. Biochem., vol. 108, pp. 379-381 (1990); Lee et al., Arch. Biochem. Biophys, vol. 291, pp. 255-257 (1991); Chang et al, J. Mol. Biol., vol. 229, pp. 235-238 (1993); Mizuno et al., J. Mol. Biol., vol. 234, pp. 1282-1283 (1993)), there is only a published structure for *Bacillus licheniformis* α-amylase (Machius et al., J. Mol. Biol. vol. 246, pp. 545-549 (1995)). However, several researchers have predicted common super-secondary structures between glucanases (MacGregor et al., Biochem. J., vol. 259, pp. 145-152 (1989)) and within α-amylases and other starch-metabolising enzymes (Jaspersen, J. Prot. Chem. vol. 12, pp. 791-805 (1993); MacGregor, Starve, vol. 45, pp. 232-237 (1993)); and sequence similarities between enzymes with similar super-secondary structures to α-amylases (Janecek, FEBS Letters, vol. 316, pp. 23-26 (1993); Janecek et al., J. Prot. Chem., vol. 12, pp. 509-514 (1993)). A structure for the *Bacillus stearothermophilus* enzyme has been modeled on that of Taka-amylase A (Holm et al., Protein Engineering, vol. 3, pp. 181-191 (1890)). The four highly conserved regions shown in Figure 7 contain many residues thought to be part of the active-site (Matsuura et al., J. Biochem. (Tokyo), vol. 95, pp. 697-702 (1984); Buisson et al., EMBO Journal, vol. 6, pp. 3909-3916 (1987); Vihinen et al., J. Biochem., vol. 107, pp. 267-272 (1990)) including His +105; Arg +229; Asp +231; His +235; Glu +261 and Asp +328 under the *Bacillus licheniformis* numbering system. The crystal structure of a bacterial amylase hybrid enzyme has been published in PCT Publication No. WO 96/23874.

As described above, the α-amylases from *Bacillus licheniformis, Bacillus stearothemiophilus, Bacillus amyloliquefaciens* and *Bacillus subtilis* all bear a significant degree of homology. However, it is known that under the conditions of industrial starch liquefaction, e.g., high temperature (excess of 90°C), low pH (pH 4-6) and low calcium, α-amylase derived from *Bacillus licheniformis* provides the most acceptable performance. Nonetheless, even *Bacillus licheniformis* α-amylase is susceptible to undesirable instability under liquefaction conditions making a more stable alternative desirable. Accordingly, much work has been performed on the *Bacillus licheniformis* derived molecule for the purpose of introducing into this enzyme properties which are desirable in connection with its use in liquefaction processes. By aligning the sequence of α-amylase from *Bacillus licheniformis* against that of α-amylase from either *Bacillus stearothermophilus* or *Bacillus amyloliquefaciens* it is possible to identify residues which differ between the homologs. According to the present invention, then, the positions at which residues differ in α-amylase from *B. stearothermophilus* or *B. amyloliquefaciens* compared to α-amylase from *B. licheniformis* are selected for substitution in α-amylase from *B. licheniformis*. The substituted residue corresponds to a position in which the same residue exists in both α-amylase from *B. stearothermophilus* and *B. amyloliquefaciens*.

Residues specifically identified herein for replacement in *Bacillus licheniformis* are those which differ from residues in a corresponding position in *Bacillus amyloliquefaciens* and/or *Bacillus stearothermophilus,* and particularly A33, A52, S85, N96, H133, S148, A209, A269, A379 and A435. While specific preferred replacements for these residues are selected from those present in both *Bacillus amyloliquefaciens* and *Bacillus stearothermophilus* and correspond to A33S, A52S, N96O, H133Y, S148N, A209V, A269K, A379S and/or A435S. It has also been discovered that the A85D mutation, which is recruited only from *Bacillus amyloliquefaciens* also provides a stability benefit. The above residues may be altered in combination with other modifications which provide a performance benefit. In a most preferred embodiment according to the invention, the above residues are in combination with mutations at a residue corresponding to any of M15, N188 and/or M197 and particularly at M15T, N188S, and/or M197T in *Bacillus licheniformis*. Any of the modifications identified herein as in accordance with the invention in combination with a mutant corresponding to M15T/H133Y/N188S/A209V in *Bacillus licheniformis* will have a particularly improved stability profile. Modifications corresponding to M15T/A33S/H133Y /N188S/A209V; M15T/D28N/A33S/H133Y/N188S/A209V; M15T/A52S /H133Y/N 188S/A209V; M15T/A52S/H133Y/N188S/A209V/L230F; M15T/S85D/H133Y/N188S/A209V; M15T/N96Q/H133Y/N188S/A209V; M15T/N96Q/H133Y/N188S/A209V/I479T; M15T/H133Y/S148N/N188S/A209V; M15T/H133Y/S148N/N188S/A209V/A379S; M15T/H133Y/S148N/N188S/A209V/G433D; M15T/H133Y/N188S/A209V/A379S; M15T/H133Y/N188S/A209V/A210S/T322A/A379S in *Bacillus licheniformis* are the invention.

The improved α-amylases according to the present invention exhibit improved performance characteristics which make those proteins particularly useful in various applications for which the proteins are commonly used and for which improved stability is desired. For example, α-amylases, according to the present invention will exhibit improved thermostability, improved pH stability and/or improved oxidative stability. Enhanced thermostability will be useful in extending the shelf life of products which incorporate them and for applications at high temperatures. Enhanced oxidative stability or improved performance is particularly desirable in cleaning products, and for extending the shelf life of the enzyme in the presence of bleach, perborate, percarbonate or peracids used in such cleaning products. An α-amylase of the present invention is especially useful in starch processing and particularly in starch liquefaction wherein oxidative and thermal stability are particularly important.

An additional embodiment of the present invention may be DNA encoding a protein according to the present invention and expression vectors comprising such DNA. The DNA sequences may be expressed by operably linking them to an expression control sequence in an appropriate expression vector and employing that expression vector to transform an appropriate host according to well known techniques. A wide variety of host/expression vector combinations may be employed in expressing the DNA sequences of this invention. Useful expression vectors, for example, include segments of chromosomal, non-chromosomal and synthetic DNA sequences, such as the various known plasmids and phages useful for this purpose. In addition, any of a wide variety of expression control sequences are generally used in these vectors. For example, with α-amylase derived from *Bacillus*, Applicants have discovered that a preferred expression control sequence for *Bacillus* transformants is the *aprE* signal peptide derived from *Bacillus subtilis*.

A wide variety of host cells are also useful in expressing the DNA sequences of this invention. These hosts may include well known eukaryotic and prokaryotic hosts, such as strains of *E. coli, Pseudomonas, Bacillus, Streptomyces*, various fungi, yeast and animal cells. Preferably, the host expresses the protein of the present invention extracellularly to facilitate purification and downstream processing. Expression and purification of the improved protein of the invention may be effected through art-recognized means for carrying out such processes.

The following is presented by way of example and is not to be construed as a limitation to the scope of the claims. Abbreviations used herein, particularly three letter or one letter notations for amino acids are described in Dale, J.W., Molecular Genetics of Bacteria, John Wiley & Sons, (1989) Appendix B.

## EXAMPLES

### EXAMPLE 1

#### Construction Of Plasmid pHP.BL

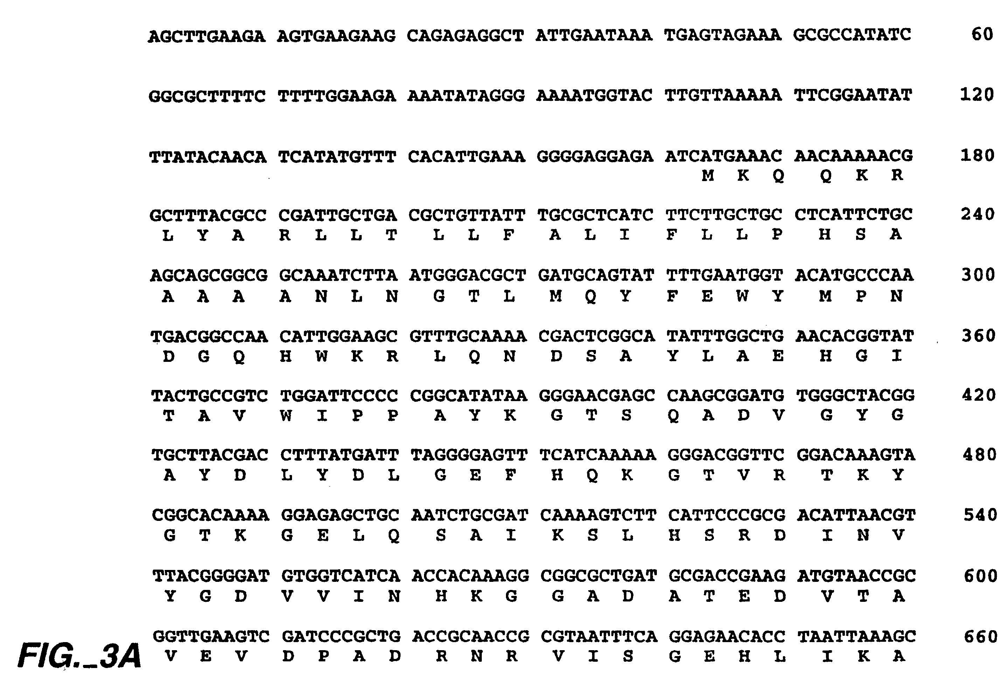

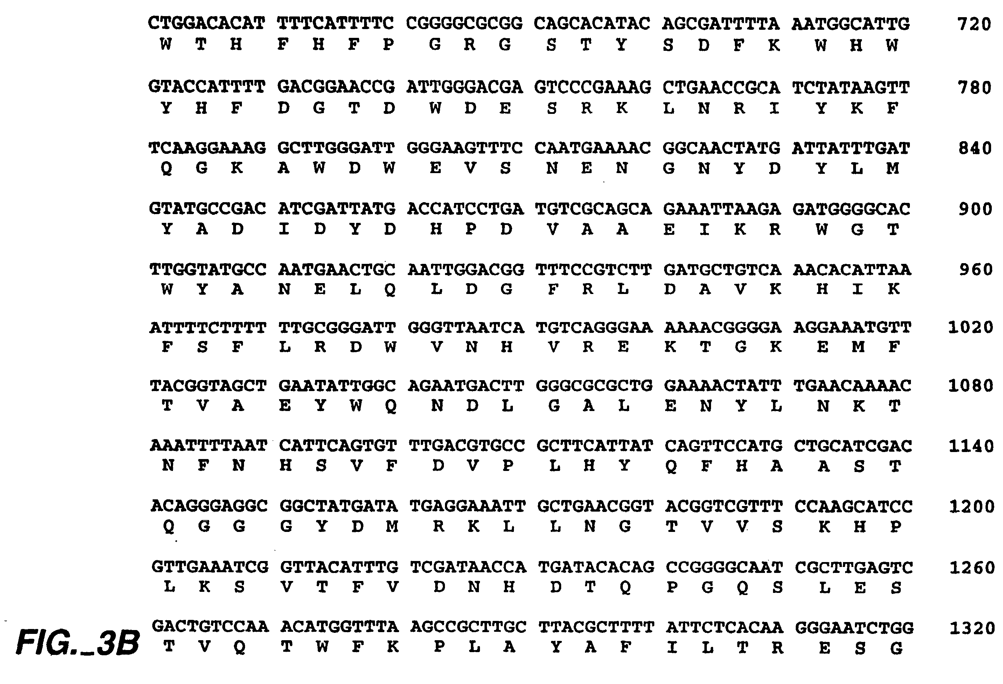

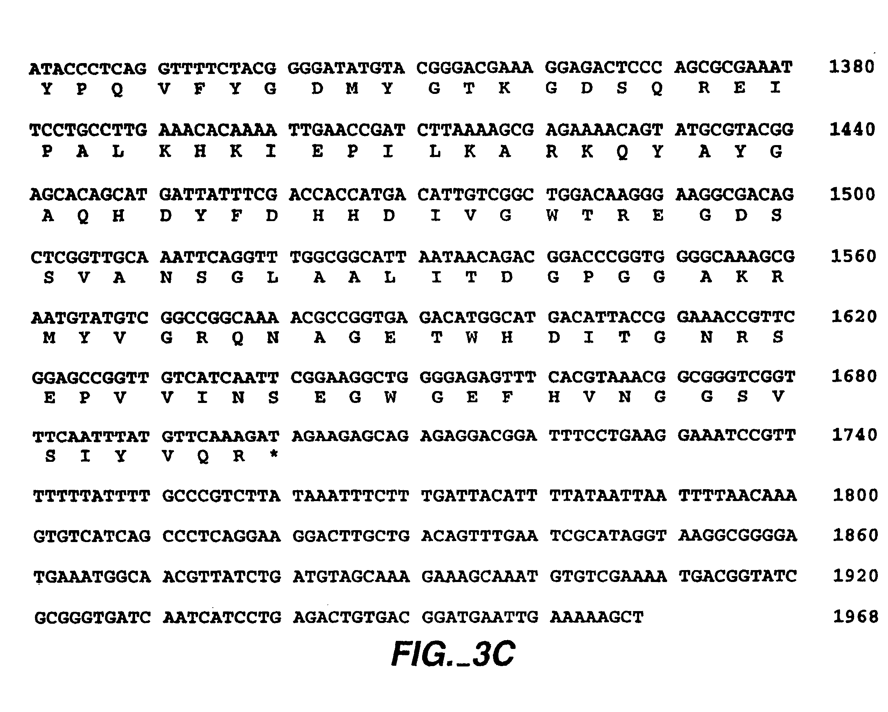

The α-amylase gene shown in Figure 3 was cloned from *Bacillus licheniformis* NCIB8061 (Gray et al., J. Bacteriology, vol. 166, pp. 635-643 (1986)). The 1.72kb PstI-SstI fragment, encoding the last three residues of the signal sequence, the entire mature protein and the terminator region, was subcloned into M13mp18. A synthetic terminator was added between the BclI and SstI sites using a synthetic oligonucleotide cassette of the form:

```
BclI                                                        SstI
5'-GATCAAAACATAAAAAACCGGCCTTGGCCCCGCCGGTTTTTTATTATTTTTGAGCT-3'   (SEQ ID NO:1)

3'     TTTTGTATTTTTTGGCCGGAACCGGGGCGGCCAAAAAATAATAAAAAC       5'   (SEQ ID NO:2)
```

designed to contain the *Bacillus amyloliquefaciens* subtilisin transcriptional terminator (Wells et al., Nucleic Acid Research, vol. 11, pp. 7911-7925 (1983)).

The pBLapr plasmid was constructed carrying the gene for the *Bacillus licheniformis* α-amylase. As illustrated in Figure 7, pBLapr comprises a 6.1kb plasmid including the ampicillin resistance gene from pBR322 and the chloramphenicol resistance gene from pC194, the aprE promoter and the gene encoding for the *Bacillus licheniformis* α-amylase ("BL AA"). The aprE promoter was constructed from a 660bp HindIII-PstI fragment encoding for the promoter and signal sequence of the *Bacillus subtilis* alkaline protease. The PstI site was removed, and an SfiI site added close to the *aprE*/BL AA junction. The BL AA gene comprises the 1720 bp PstI-SstI fragment described above. In the work described herein, pBLapr was constructed with an SfiI site adjacent to the 5' end of the start of the coding sequence for the mature amylase gene. Specifically, the 5' end of the pBLapr construction was subcloned on an EcoRI-SstII fragment from pBLapr into M13BM20 (Boehringer Mannheim) to obtain a coding-strand template for the mutagenic oligonucleotide below:

5'- CCC ATT AAG ATT <u>GGC CGC CTG GGC C</u>GA CAT GTT GCT GG - 3' (SEQ ID NO:3)

This primer introduced an SfiI site (indicated by underlining) which allowed correct forms to be screened for by the presence of this unique restriction site. Subcloning the EcoRI-SstII fragment back into the pBLapr vector gave a version of the plasmid containing an SfiI site.

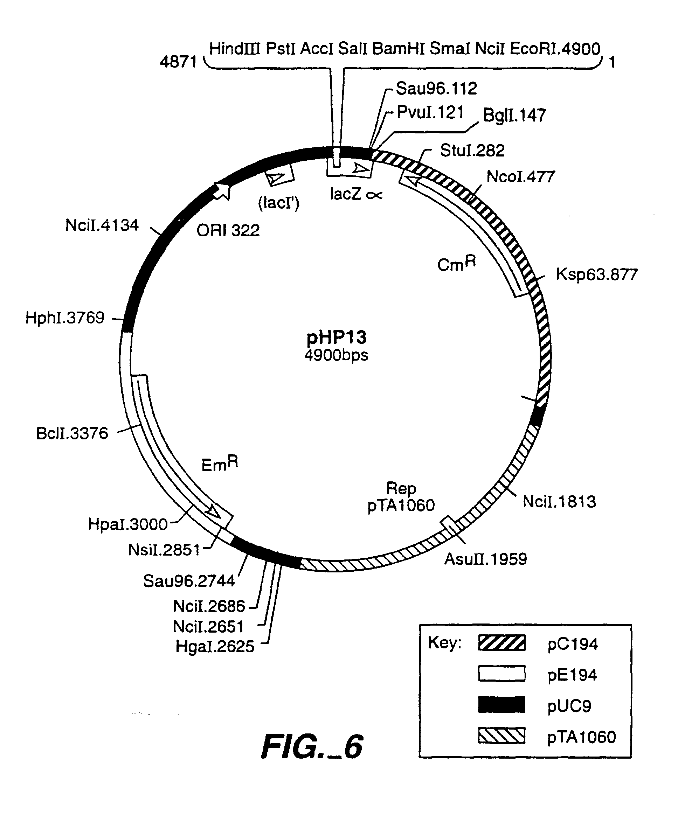

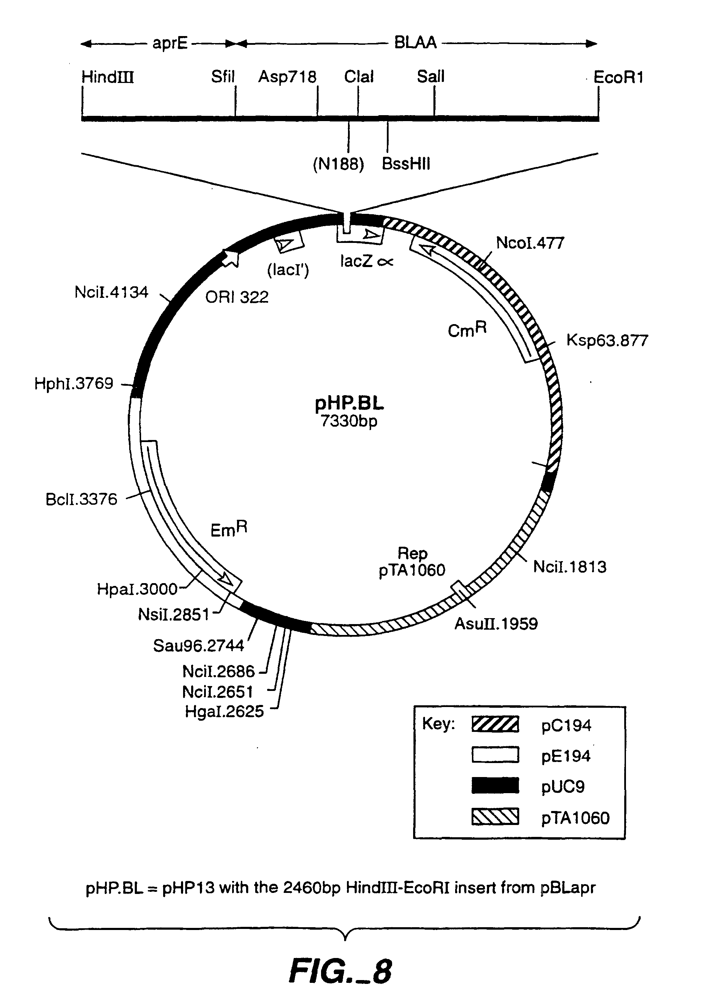

Plasmid pHP13 (Haima et al., Mol. Gen. Genet., vol. 209, pp. 335-342 (1987)) (Figure 6) was digested with restriction enzymes EcoRI and HindIII and the resulting vector purified on a polyacrymide gel and then eluted. Plasmid pBLapr was digested with HindIII, Asp718 and in a separate incubation with Asp718, EcoRI and gel purified. Two bands, HindIII-Asp718 (1203 bp) and Asp718-EcoRI (1253 bp)were gel purified, eluted from the gel and ligated into the vector by a 3-way ligation, to give plasmid pHP.BL, the plasmid used in expression of the α-amylase (Figure 8).

<u>EXAMPLE 2</u>

<u>Construction Of Plasmid Encoding α-Amylase</u>

<u>Comprising Substitutions For Asparagine 188</u>

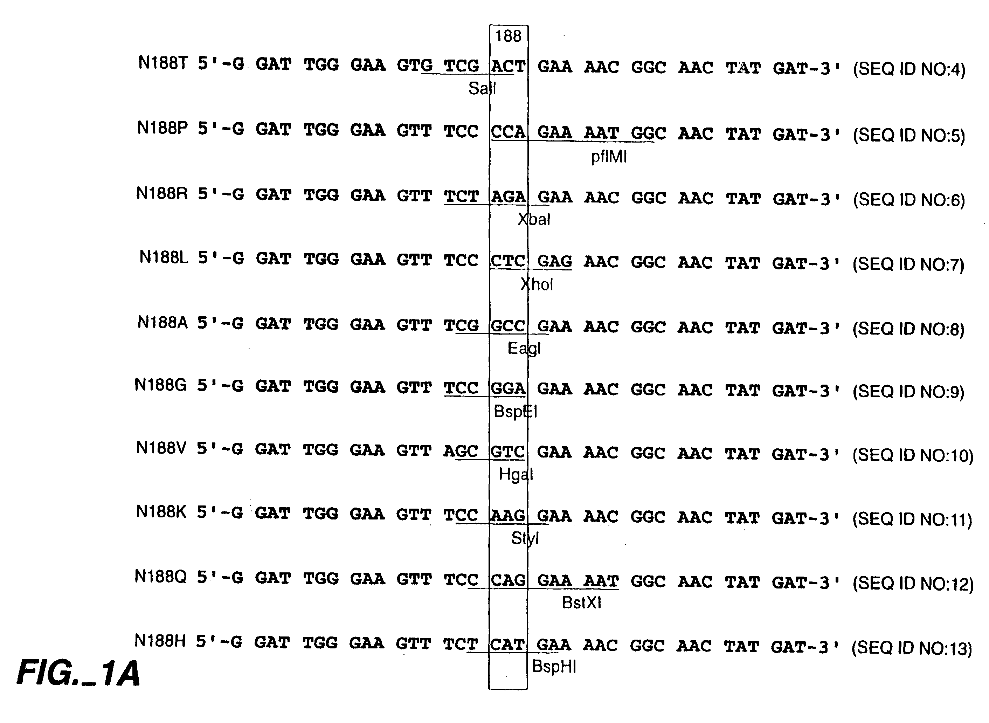

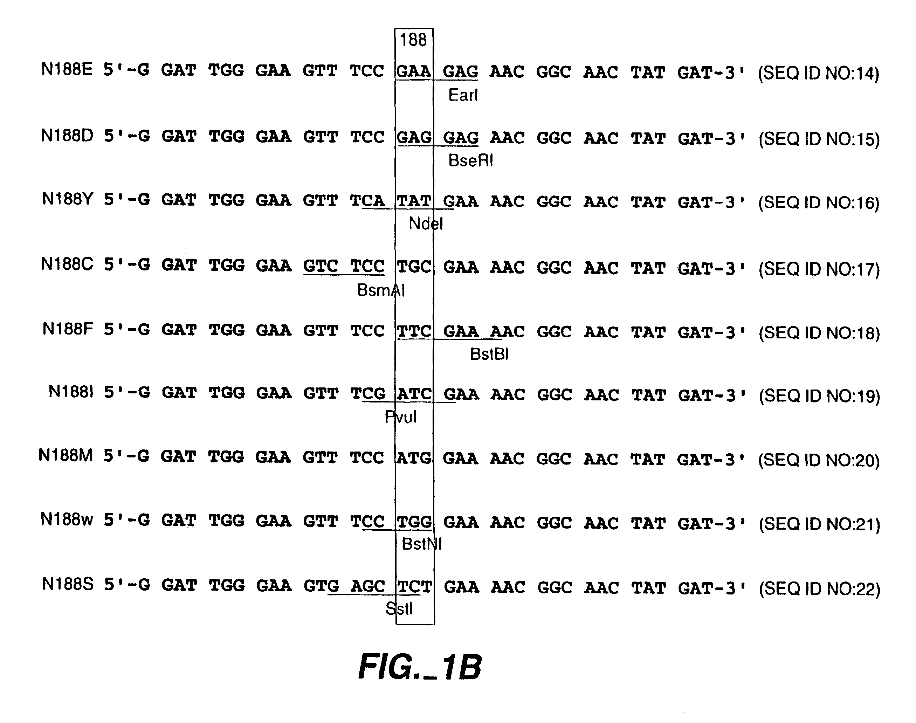

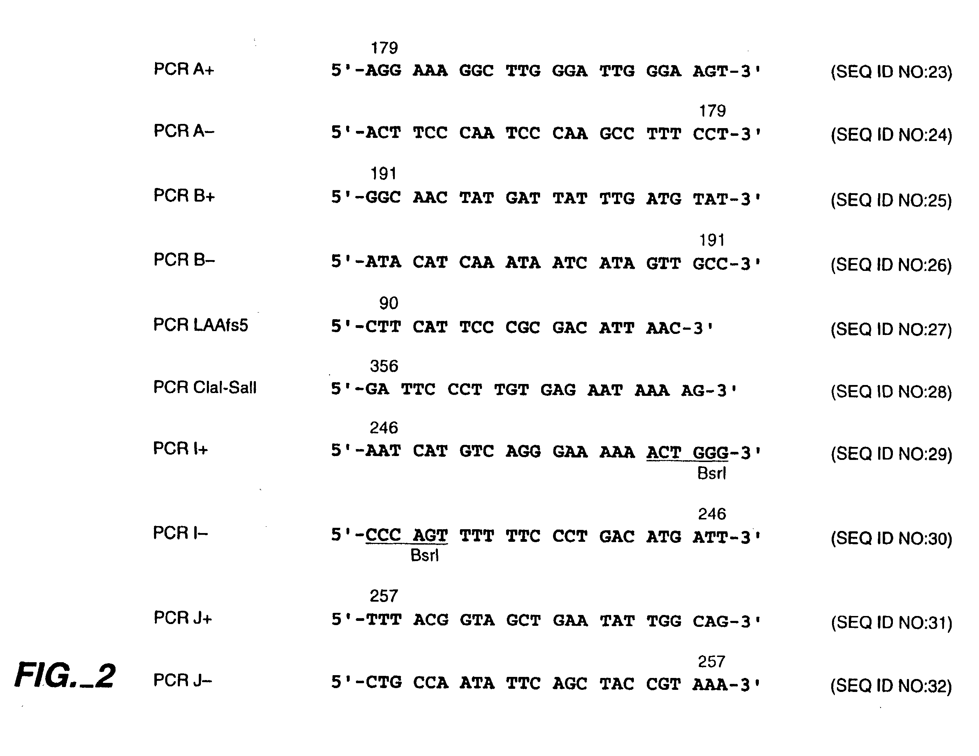

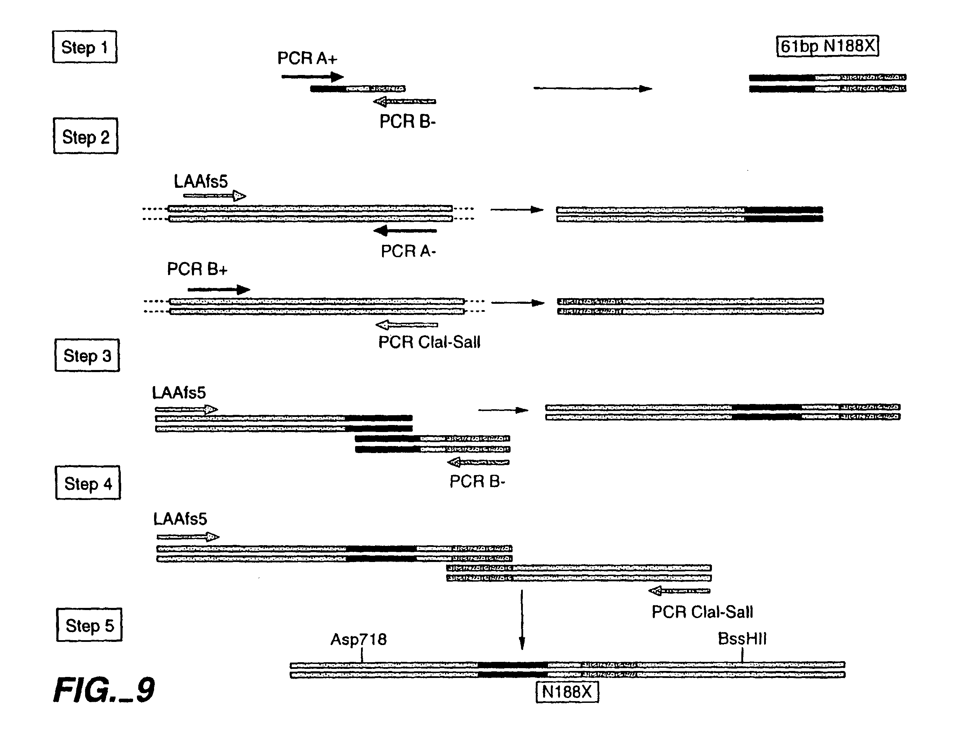

A series of mutagenic primers encoding for substitutions of Asn188 ("N188") with each of the naturally occurring amino acids were synthesized and are shown in Figure 1 (SEQ ID NOS:4-22). The α-amylase gene mutations encoding for these changes were made by PCR, according to the procedure summarized in Figure 9, using the PCR primers shown in Figure 2 (SEQ ID NOS:23-32).

Step (1): The mutagenic primers were used as templates for the PCR primers PCR A+ and PCR B- resulting in a lengthened (61 bp) double stranded DNA. Each contained a different amino acid replacement at position 188, and all except N188M contained a different restriction site. Initially the PCR primers were annealed at 35°C for five minutes followed by a one minute DNA extension with taq polymerase at 75°C. The double stranded DNA was then melted at 95°C for one minute, followed by the annealing and extension steps. Melting, annealing and extension continued for a total of 30 cycles.

Step (2): DNA upstream and downstream of position 188 were made in separate PCR reactions. The template was pBLapr, and the PCR primers were LAAfs5 (SEQ ID NO:27) and PCR A- (SEQ ID NO:24) for upstream; and PCR B+ (SEQ ID NO:25) and PCR Cla-SalI (SEQ ID NO:28) for downstream DNA. The DNA was melted at 95°C for one minute, annealed at 45°C for three minutes and elongated at 68°C for 3 minutes. The upstream portion is 290 bp and downstream is 498 bp. This procedure was repeated for 18 cycles using pfu polymerase. The same PCR procedure was used in steps (3) and (4).

Step (3): The upstream portion of DNA described in step (2) was attached to the double stranded mutagenic primers described in step (1). Primers LAAfs5 (SEQ ID NO:27) and PCR B- (SEQ ID NO:26) were used. As the result of primer design there is a 24 bp overlap between these templates allowing for the attachment of the two pieces of DNA.

Step (4): The downstream portions of DNA described in Step (2) and the product of Step (3) were attached to give the final product. A 24 bp overlap between the two PCR products allows for the attachment. Primers used were LAAfs5 (SEQ ID NO:27) and PCR ClaI-SalI (SEQ ID NO:28).

Step (5): Unique restriction sites, Asp718 and BssHII, are located upstream and downstream, respectively, of the 188 site. The final PCR product is digested with Asp718 and BssHII, the 333 bp fragment isolated by polyacrylamide gel electrophoresis and subcloned into the pHP.BL vector to obtain pHP.N188X.

Mutations were confirmed by dideoxy sequencing (Sanger et al., Proc. Natl. Acad. Sci. U.S.A., vol. 74, pp. 5463-5467 (1977)).

With reference to the DNA sequence and numbering system used in Figure 3, the codon encoding for the +188 amino acid position is at base pairs 812-814. PCR primers A+ and A- correspond to base pairs 784-807. PCR primers B+ and B- correspond to base pairs 821-844. The 5' end of PCR primer LAAfs5 corresponds to base pair 518. The 5' end of PCR primer PCR ClaI-SalI corresponds to base pair 1317. The Asp718 site corresponds to base pair 724. The BssHII site corresponds to base pair 1053.

EXAMPLE 3

Construction Of Plasmid Encoding Mutations At M15 And N188

A pBLapr plasmid having threonine substituted for methionine at amino acid 15 was constructed according to U.S. Patent Application Serial No. 08/194,664 (PCT Publication No. WO 94/18314). This plasmid (pBLaprM15T) was digested with SfiI and Asp718, and the 477 base pair fragment subcloned into pHP.BL to create pHP.M15T. In a manner analogous to that described above, Example 1, pHP.M15T was digested with Asp718 and BssHII, gel purified and eluted from the gel. The 333 base pair fragment comprising Asp718 to BssHII and the fragment from pHP.N188S were then subcloned into pHP.M15T to give plasmid pHP.M15T/N188S. In an analogous manner, starting with plasmids pBL aprM15L and pHP.N188Y, the plasmid pHP.M15L/N188Y was constructed.

EXAMPLE 4

Transformation Of Plasmids Into *Bacillus subtilis*, Expression And Purification of Mutant α-Amylase

α-Amylase was expressed in *Bacillus subtilis* after transformation with the plasmids described in Examples 1-3. pHP13 is a plasmid able to replicate in *E. coli* and in *Bacillus subtilis.* Plasmids containing different variants were constructed using *E. coli* strain MM294, the plasmids isolated and then transformed into *Bacillus subtilis* as described in Anagnostopoulos et at., J. Bacter., vol. 81, pp. 741-746 (1961). The *Bacillus* strain had been deleted for two proteases (Δapr, Δnpr) (see e.g., Ferrari et al., U.S. Patent No. 5,264,366) and for amylase (Δ*amyE*) (see e.g., Stahl et al., J. Bacter., vol. 158, pp. 411-418 (1984)). The *bacillus* strain expressing M15L/N188Y was found to form larger zones of clearing than the strain expressing M15L on agar plates containing 1% insoluble starch indicating increased amylolytic activity. After transformation, the sacU(Hy) mutation (Henner et al., J. Bacter., vol., 170, pp. 296-300 (1988)) was introduced by PBS-1 mediated transduction (Hoch, J. Bact., vol. 154, pp. 1513-1515 (1983)).

Secreted amylases were routinely recovered from *Bacillus subtilis* cultures as follows: The culture supernatant was adjusted to 20% saturated ammonium sulfate and stirred for one hr. at 4°C. After centrifugation, the resultant supernatant was adjusted to 70% saturated ammonium sulfate and stirred for one hr. at 4°C. After centrifugation of the supernatant, the resultant pellet was re-dissolved in 50mM sodium acetate, pH 6.0, 5mM calcium chloride, and sterile filtered.

EXAMPLE 5

Assay For Determining α-Amylase Activity

Soluble Substrate Assay: A rate assay was developed based on an end-point assay kit supplied by Megazyme (Aust.) Pty. Ltd. A vial of substrate (*p*-nitrophenyl maltoheptaoside, BPNPG7) was dissolved in 10ml of sterile water followed by a 1:4 dilution in assay buffer (50mM maleate buffer, pH 6.7, 5mM calcium chloride, 0.002% Tween20). Assays were performed by adding 10μl of amylase to 790μl of the substrate in a cuvette at 25°C. Rates of hydrolysis were measured as the rate of change of absorbance at 410nm, after a delay of 75 seconds. The assay was linear up to rates of 0.2 absorption units/min.

α-Amylase protein concentration was measured using the standard Bio-Rad Assay (Bio-Rad Laboratories) based on the method of Bradford, Anal. Biochem., vol. 72, p. 248 (1976) using bovine serum albumin standards.

Starch Hydrolysis Assay: α-Amylase activity on starch was determined through an assay which depends on the ability of starch to form a blue colored complex with iodine and the disappearance of this color when starch is hydrolyzed to shorter dextrin molecules. The α-amylase activity was defined in terms of the digestion time required to produce a color change denoting a definite state of dextrination of the starch.

Reagents used were as follows:

*Phosphate buffer* - Potassium dihydrogen phosphate (340 g) and sodium hydroxide (25.3 g) were dissolved in water and diluted to - two liters. The buffer was cooled to room temperature and the pH was adjusted to 6.2±0.1. The buffer was diluted to two liters in a volumetric flask.

*Starch substrate* - Ten grams (dry substance) of soluble lintner starch were suspended in 50 ml of water and washed into -300 ml of boiling water. The suspension was again brought to boiling and was boiled for five minutes with constant stirring. The starch solution was cooled with constant stirring to room temperature and 125 ml of phosphate buffer was added. The solution was diluted to 500 ml with water. The starch substrate was made fresh daily.

*Stock iodine solution* - crystals (5.5 g) and potassium iodide (11.0 g) were dissolved in water and were volumetrically diluted to 250 ml. The solution was kept from light.

*Dilute iodine solution* - Potassium iodide (20 g) and two ml of stock iodine solution were dissolved in water and diluted volumetrically to 500 ml. The solution was made fresh daily.

*Enzyme diluting solution* - Calcium chloride (11.1 g) was dissolved in four liters of water. Water used for all reagents was either distilled or deionized.

An α-amylase sample was diluted to between 10-15 LU/ml (as defined below) with enzyme diluting solution. For many commercial α-amylase preparations a suitable dilution was found to be 2000 fold. Five milliliter aliquots of dilute iodine solution were dispensed into 13 x 100 mm test tubes and 10 ml of starch substrate was placed in a 23 x 200 mm test tube. All tubes were placed in the 30°C water bath. A Hellige comparator equipped with a special α-amylase color disc (catalog number 620-s5) was used to make readings. Five milliliters of diluted enzyme (also at 30°C) were mixed with the starch substrate and timing was begun. At appropriate time intervals, for example one minute intervals early in the reaction and 15 second intervals later in the reaction, one ml aliquots of the enzyme-substrate mixture were transferred to a tube containing the dilute iodine solution. The starch iodine solution was mixed and transferred to a 13 mm precision square tube and the color was compared with the standard α-amylase color disc in the Hellige comparator. When the time of the end point was approached, samples were taken at 0.25 minute intervals.

The time required for the colors of the samples and the color disc to match were recorded and the activity (in liquefons per gram or ml) was calculated according to the formula:

$$LU/ml \text{ or } LU/g =$$

Where:

| | |
|---|---|
| LU = | liquefon unit |
| V = | volume of enzyme (5 ml or grams) |
| t = | dextrinization time (minutes) |
| D = | dilution factor:dilution volume divided by ml or g of enzyme diluted. |

## EXAMPLE 6

### Preparation and Testing of Additional Mutant Alpha-Amylases for Thermal Stability

Mutant alpha-amylases were prepared having substitutions at one or more of five positions for which corresponding residues in both *Bacillus stearothermophilus* and *Bacillus amyloliquefaciens* were identical: A33S, A52S, N96Q S148N, A379S in combination with M15T/H133Y/N1885/A209V and compared with a mutant comprising only the M15T/H133Y/N1885/A209V substitutions. Additionally, the mutation S85D which represents a recruitment from the *Bacillus* amyloliquefaciens. The mutations were prepared according to the procedures provided in Examples 1-4 except that appropriate PCR primers were provided to effect the desired mutations.

Thermal inactivation rates for the various mutants were measured according to the following procedure. Amylase stock solutions were dialysed extensively into 20 mM ammonium acetate, 4 mM $CaCl_2$ pH 6.5. For measurement of stability, this stock was diluted >50fold into a solution designed to induce rapid inactivation of wild type amylase: 50mM ammonium acetate, 5mM $CaCl_2$, 0.02% Tween 20 and a pH of 4.9, or 4.8 to a final concentration of between 30 and 50 μg/ml. Six 100μl aliquots were put into Eppendorf tubes and placed into a water bath at either 82°C or 83°C. The Eppendorf tubes were removed at regular, measured intervals of between 30 seconds and 5 minutes and placed on ice to stop the inactivation. The residual activity was assayed using a soluble substrate as described in Example 5. The natural log of the activity was plotted against time of incubation, and the rate constant for inactivation obtained from the slope of the straight line. The half-life was calculated as *ln*(2) divided by the rate constant. Results for various mutants are provided in Tables 1-4.

TABLE 1

| pH 4.9, 83°C | | |
|---|---|---|
| Variant | Inactivation rate constant ($Min^{-1}$) | $t_{1/2}$ (min.) |
| M15T/H133Y/N188S/A209V | 0.171 | 4.05 |
| M15T/D28N/A33S/H133Y/N188S/A209V | 0.137 | 5.06 |
| M15T/A52S/H133Y/N188S/A209V/L230F | 0.144 | 4.81 |
| M15T/N96Q/H133Y/N188S/A209V/1479T | 0.162 | 4.27 |
| M15T/H133Y/S148N/N188S/A209V/G433D | 0.121 | 5.73 |
| M15T/H133Y/N188S/A209V/A379S | 0.145 | 4.78 |

TABLE 2

| pH 4.85, 83°C | | |
|---|---|---|
| Variant | Inactivation rate constant ($Min^{-1}$) | $t_{1/2}$ (min.) |
| M15T/H133Y/N188S/A209V | 0.252 | 2.75 |
| M15T/H133Y/N188S/A209V | 0.235 | 2.95 |
| M15T/H133Y/S148N/N188S/A209V/A379S | 0.114 | 6.05 |

TABLE 3

| pH4.85, 82°C | | |
|---|---|---|
| Variant | Inactivation rate constant ($Min^{-1}$) | $t_{1/2}$ (min.) |
| M15T/H133Y/N188S/A209V | 0.203 | 3.41 |
| M15T/H133Y/S148N/N188S/A209V/A379S | 0.106 | 6.54 |
| M15T/H133Y/N188S/A209V/A210S/T322A/A379S | 0.141 | 4.89 |
| M15T/H133Y/S148N/N188S/A209V | 0.122 | 5.65 |

TABLE 4

| pH4.80, 82.2°C | | |
|---|---|---|
| Variant | Inactivation rate constant ($Min^{-1}$) | $t_{1/2}$ (min.) |
| Wild Type | >2.0 | <0.35 |
| M15T/N188S | >1.9 | <0.36 |
| M15T/H133Y/N188S/A209V | 0.267 | 2.59 |
| M15T/S85D/H133Y/N188S/A209V | 0.236 | 2.93 |

**Claims**

**1.** An improved α-amylase comprising an amino acid sequence which has been modified from a precursor amino acid sequence corresponding to α-amylase from *Bacillus licheniformis* by the substitution of amino acid residues, said substitutions comprising
M15T/A33S/H133Y/N188S/A209V, M15T/D28N/A33S/H133Y/N188S/A209V,
M15T/A52S/H133Y/N188S/A209V,
M15T/A52S/H133Y/N188S/A209V/L230F,
M15T/S85D/H133Y/N188S/A209V, M15T/N96Q/H133Y/N188S/A209V,

M15T/N96Q/H133Y/N188S/A209V/I479T,
M15T/H133Y/S148N/N188S/A209V,
M15T/H133Y/S148N/N188S/A209V/A379S,
M15T/H133Y/S148N/N188S/A209V/G433D,
M15T/H133Y/N188S/A209V/A379S, and/or
M15T/H133Y/N188S/A209V/A210S/T322A/A379S,
wherein said improved protein has altered stability compared to a protein corresponding to the precursor amino acid sequence.

**2.** The improved α-amylase of claim 1, comprising the substitutions M15T/H133Y/S148N/N188S/A209V.

**3.** The improved α-amylase of claim 1 or claim 2, comprising the substitutions M15T/H133Y/S148N/N188S/A209V/A379S.

**4.** The improved α-amylase of claim 1 or claim 2, comprising the substitutions M15T/H133Y/S148N/N188S/A209V/G433D.

**5.** A method for the liquefaction of starch comprising the steps of:

(a) preparing an aqueous solution of starch; and
(b) contacting said aqueous solution of starch with the a-amylase of any one of the preceding claims.

**Patentansprüche**

**1.** Verbesserte α-Amylase, welche eine Aminosäuresequenz umfasst, die ausgehend von einer Vorläufer-Aminosäuresequenz, welche der α-Amylase aus *Bacillus licheniformis* entspricht, durch die Substitution von Aminosäurenresten modifiziert worden ist, wobei diese Substitutionen
M15T/A33S/H133Y/N188S/A209V, M15T/D28N/A33S/H133Y/N188S/A209V,
M15T/A52S/H133Y/N188S/A209V,
M15T/A52S/H133Y/N188S/A209V/L230F,
M15T/S85D/H133Y/N188S/A209V, M15T/N96Q/H133Y/N188S/A209V,
M15T/N96Q/H133Y/N188S/A209V/I479T,
M15T/H133Y/S148N/N188S/A209V,
M15T/H133Y/S148N/N188S/A209V/A379S,
M15T/H133Y/S148N/N188S/A209V/G433D,
M15T/H133Y/N188S/A209V/A379S, und/oder
M15T/H133Y/N188S/A209V/A210S/T322A/A379S,
umfassen, wobei das verbesserte Protein eine verglichen mit einem Protein, welches der Vorläufer-Aminosäuresequenz entspricht, veränderte Stabilität aufweist.

**2.** Verbesserte α-Amylase nach Anspruch 1, welche die Substitutionen M15T/H133Y/S148N/N188S/A209V umfasst.

**3.** Verbesserte α-Amylase nach Anspruch 1 oder Anspruch 2, welche die Substitutionen M15T/H133Y/S148N/N188S/A209V/A379S umfasst.

**4.** Verbesserte α-Amylase nach Anspruch 1 oder Anspruch 2, welche die Substitutionen M15T/H133Y/S148N/N188S/A209V/G433D umfasst.

**5.** Verfahren zur Verflüssigung von Stärke, welches die Schritte umfasst:

(a) eine wässrige Stärkelösung herzustellen; und
(b) die wässrige Stärkelösung mit der α-Amylase nach einem der vorangegangenen Ansprüche in Kontakt zu bringen.

## Revendications

1. α-amylase améliorée comprenant une séquence d'acides aminés qui a été modifiée à partir d'une séquence d'acides aminés précurseur correspondant à une α-amylase de *Bacillus licheniformis* par la substitution de résidus d'acides aminés, lesdites substitutions comprenant
   M15T/A33S/H133Y/N188S/A209V, M15T/D28N/A33S/HL33Y/N188S/A209V, M15T/A52S/H133Y/N188S/A209V,
   M15T/A52S/H133Y/N188S/A209V/L230F,
   M15T/S85D/H133Y/N188S/A209V, M15T/N96Q/H133Y/N188S/A209V,
   M15T/N96Q/H133Y/N188S/A209V/I479T,
   M15T/H133Y/S148N/N188S/A209V,
   M15T/H133Y/S148N/N188S/A209V/A379S,
   M15T/H133Y/S148N/N188S/A209V/G433D,
   M15T/H133Y/N188S/A209V/A379S, et/ou
   M15T/H133Y/N188S/A209V/A210S/T322A/A379S,
   dans laquelle ladite protéine améliorée a une stabilité modifiée comparée à une protéine correspondant à la séquence d'acides aminés précurseur.

2. α-amylase améliorée de la revendication 1, comprenant les substitutions M15T/H133Y/S148N/N188S/A209V.

3. α-amylase améliorée de la revendication 1 ou de la revendication 2, comprenant les substitutions M15T/H133Y/S148N/N188S/A209V/A379S.

4. α-amylase améliorée de la revendication 1 ou de la revendication 2, comprenant les substitutions M15T/H133Y/S148N/N188S/A209V/G433D.

5. Procédé de liquéfaction d'amidon comprenant les étapes de :

   (a) préparation d'une solution aqueuse d'amidon ; et
   (b) mise en contact de ladite solution aqueuse d'amidon avec l'α-amylase de l'une quelconque des revendications précédentes.

188

N188T 5'-G GAT TGG GAA GTG TCG ACT GAA AAC GGC AAC TAT GAT-3' (SEQ ID NO:4)
Sall

N188P 5'-G GAT TGG GAA GTT TCC CCA GAA AAT GGC AAC TAT GAT-3' (SEQ ID NO:5)
pflMI

N188R 5'-G GAT TGG GAA GTT TCT AGA GAA AAC GGC AAC TAT GAT-3' (SEQ ID NO:6)
Xbal

N188L 5'-G GAT TGG GAA GTT TCC CTC GAG AAC GGC AAC TAT GAT-3' (SEQ ID NO:7)
Xhol

N188A 5'-G GAT TGG GAA GTT TCG GCC GAA AAC GGC AAC TAT GAT-3' (SEQ ID NO:8)
Eagl

N188G 5'-G GAT TGG GAA GTT TCC GGA GAA AAC GGC AAC TAT GAT-3' (SEQ ID NO:9)
BspEI

N188V 5'-G GAT TGG GAA GTT AGC GTC GAA AAC GGC AAC TAT GAT-3' (SEQ ID NO:10)
Hgal

N188K 5'-G GAT TGG GAA GTT TCC AAG GAA AAC GGC AAC TAT GAT-3' (SEQ ID NO:11)
Styl

N188Q 5'-G GAT TGG GAA GTT TCC CAG GAA AAT GGC AAC TAT GAT-3' (SEQ ID NO:12)
BstXI

N188H 5'-G GAT TGG GAA GTT TCT CAT GAA AAC GGC AAC TAT GAT-3' (SEQ ID NO:13)
BspHI

FIG._1A

188

N188E 5'-G GAT TGG GAA GTT TCC GAA GAG AAC GGC AAC TAT GAT-3' (SEQ ID NO:14)
EarI

N188D 5'-G GAT TGG GAA GTT TCC GAG GAG AAC GGC AAC TAT GAT-3' (SEQ ID NO:15)
BseRI

N188Y 5'-G GAT TGG GAA GTT TCA TAT GAA AAC GGC AAC TAT GAT-3' (SEQ ID NO:16)
NdeI

N188C 5'-G GAT TGG GAA GTC TCC TGC GAA AAC GGC AAC TAT GAT-3' (SEQ ID NO:17)
BsmAI

N188F 5'-G GAT TGG GAA GTT TCC TTC GAA AAC GGC AAC TAT GAT-3' (SEQ ID NO:18)
BstBI

N188I 5'-G GAT TGG GAA GTT TCG ATC GAA AAC GGC AAC TAT GAT-3' (SEQ ID NO:19)
PvuI

N188M 5'-G GAT TGG GAA GTT TCC ATG GAA AAC GGC AAC TAT GAT-3' (SEQ ID NO:20)

N188w 5'-G GAT TGG GAA GTT TCC TGG GAA AAC GGC AAC TAT GAT-3' (SEQ ID NO:21)
BstNI

N188S 5'-G GAT TGG GAA GTG AGC TCT GAA AAC GGC AAC TAT GAT-3' (SEQ ID NO:22)
SstI

*FIG._1B*

**FIG._2**

| Primer | Sequence | SEQ ID |
|---|---|---|
| PCR A+ | 179<br>5'-AGG AAA GGC TTG GGA TTG GGA AGT-3' | (SEQ ID NO:23) |
| PCR A– | 179<br>5'-ACT TCC CAA TCC CAA GCC TTT CCT-3' | (SEQ ID NO:24) |
| PCR B+ | 191<br>5'-GGC AAC TAT GAT TAT TTG ATG TAT-3' | (SEQ ID NO:25) |
| PCR B– | 191<br>5'-ATA CAT CAA ATA ATC ATA GTT GCC-3' | (SEQ ID NO:26) |
| PCR LAAfs5 | 90<br>5'-CTT CAT TCC CGC GAC ATT AAC-3' | (SEQ ID NO:27) |
| PCR ClaI-SalI | 356<br>5'-GA TTC CCT TGT GAG AAT AAA AG-3' | (SEQ ID NO:28) |
| PCR I+ | 246<br>5'-AAT CAT GTC AGG GAA AAA ACT GGG-3' (BsrI: ACT GGG) | (SEQ ID NO:29) |
| PCR I– | 246<br>5'-CCC AGT TTT TTC CCT GAC ATG ATT-3' (BsrI: CCC AGT) | (SEQ ID NO:30) |
| PCR J+ | 257<br>5'-TTT ACG GTA GCT GAA TAT TGG CAG-3' | (SEQ ID NO:31) |
| PCR J– | 257<br>5'-CTG CCA ATA TTC AGC TAC CGT AAA-3' | (SEQ ID NO:32) |

```
AGCTTGAAGA AGTGAAGAAG CAGAGAGGCT ATTGAATAAA TGAGTAGAAA GCGCCATATC    60

GGCGCTTTTC TTTTGGAAGA AAATATAGGG AAAATGGTAC TTGTTAAAAA TTCGGAATAT   120

TTATACAACA TCATATGTTT CACATTGAAA GGGGAGGAGA ATCATGAAAC AACAAAAACG   180
                                              M  K  Q   Q  K  R
GCTTTACGCC CGATTGCTGA CGCTGTTATT TGCGCTCATC TTCTTGCTGC CTCATTCTGC   240
 L  Y  A   R  L  L  T   L  L  F   A  L  I   F  L  L  P   H  S  A
AGCAGCGGCG GCAAATCTTA ATGGGACGCT GATGCAGTAT TTTGAATGGT ACATGCCCAA   300
 A  A  A   A  N  L  N   G  T  L   M  Q  Y   F  E  W  Y   M  P  N
TGACGGCCAA CATTGGAAGC GTTTGCAAAA CGACTCGGCA TATTTGGCTG AACACGGTAT   360
 D  G  Q   H  W  K  R   L  Q  N   D  S  A   Y  L  A  E   H  G  I
TACTGCCGTC TGGATTCCCC CGGCATATAA GGGAACGAGC CAAGCGGATG TGGGCTACGG   420
 T  A  V   W  I  P  P   A  Y  K   G  T  S   Q  A  D  V   G  Y  G
TGCTTACGAC CTTTATGATT TAGGGGAGTT TCATCAAAAA GGGACGGTTC GGACAAAGTA   480
 A  Y  D   L  Y  D  L   G  E  F   H  Q  K   G  T  V  R   T  K  Y
CGGCACAAAA GGAGAGCTGC AATCTGCGAT CAAAAGTCTT CATTCCCGCG ACATTAACGT   540
 G  T  K   G  E  L  Q   S  A  I   K  S  L   H  S  R  D   I  N  V
TTACGGGGAT GTGGTCATCA ACCACAAAGG CGGCGCTGAT GCGACCGAAG ATGTAACCGC   600
 Y  G  D   V  V  I  N   H  K  G   G  A  D   A  T  E  D   V  T  A
GGTTGAAGTC GATCCCGCTG ACCGCAACCG CGTAATTTCA GGAGAACACC TAATTAAAGC   660
 V  E  V   D  P  A  D   R  N  R   V  I  S   G  E  H  L   I  K  A
```

FIG._3A

```
CTGGACACAT TTTCATTTTC CGGGGCGCGG CAGCACATAC AGCGATTTTA AATGGCATTG   720
 W  T  H   F  H  F  P   G  R  G   S  T  Y   S  D  F  K   W  H  W

GTACCATTTT GACGGAACCG ATTGGGACGA GTCCCGAAAG CTGAACCGCA TCTATAAGTT   780
 Y  H  F   D  G  T  D   W  D  E   S  R  K   L  N  R  I   Y  K  F

TCAAGGAAAG GCTTGGGATT GGGAAGTTTC CAATGAAAAC GGCAACTATG ATTATTTGAT   840
 Q  G  K   A  W  D  W   E  V  S   N  E  N   G  N  Y  D   Y  L  M

GTATGCCGAC ATCGATTATG ACCATCCTGA TGTCGCAGCA GAAATTAAGA GATGGGGCAC   900
 Y  A  D   I  D  Y  D   H  P  D   V  A  A   E  I  K  R   W  G  T

TTGGTATGCC AATGAACTGC AATTGGACGG TTTCCGTCTT GATGCTGTCA AACACATTAA   960
 W  Y  A   N  E  L  Q   L  D  G   F  R  L   D  A  V  K   H  I  K

ATTTTCTTTT TTGCGGGATT GGGTTAATCA TGTCAGGGAA AAAACGGGGA AGGAAATGTT  1020
 F  S  F   L  R  D  W   V  N  H   V  R  E   K  T  G  K   E  M  F

TACGGTAGCT GAATATTGGC AGAATGACTT GGGCGCGCTG GAAAACTATT TGAACAAAAC  1080
 T  V  A   E  Y  W  Q   N  D  L   G  A  L   E  N  Y  L   N  K  T

AAATTTTAAT CATTCAGTGT TTGACGTGCC GCTTCATTAT CAGTTCCATG CTGCATCGAC  1140
 N  F  N   H  S  V  F   D  V  P   L  H  Y   Q  F  H  A   A  S  T

ACAGGGAGGC GGCTATGATA TGAGGAAATT GCTGAACGGT ACGGTCGTTT CCAAGCATCC  1200
 Q  G  G   G  Y  D  M   R  K  L   L  N  G   T  V  V  S   K  H  P

GTTGAAATCG GTTACATTTG TCGATAACCA TGATACACAG CCGGGGCAAT CGCTTGAGTC  1260
 L  K  S   V  T  F  V   D  N  H   D  T  Q   P  G  Q  S   L  E  S

GACTGTCCAA ACATGGTTTA AGCCGCTTGC TTACGCTTTT ATTCTCACAA GGGAATCTGG  1320
 T  V  Q   T  W  F  K   P  L  A   Y  A  F   I  L  T  R   E  S  G
```

FIG._3B

```
ATACCCTCAG GTTTTCTACG GGGATATGTA CGGGACGAAA GGAGACTCCC AGCGCGAAAT   1380
 Y  P  Q   V  F  Y  G   D  M  Y   G  T  K   G  D  S  Q   R  E  I

TCCTGCCTTG AAACACAAAA TTGAACCGAT CTTAAAAGCG AGAAAACAGT ATGCGTACGG   1440
 P  A  L   K  H  K  I   E  P  I   L  K  A   R  K  Q  Y   A  Y  G

AGCACAGCAT GATTATTTCG ACCACCATGA CATTGTCGGC TGGACAAGGG AAGGCGACAG   1500
 A  Q  H   D  Y  F  D   H  H  D   I  V  G   W  T  R  E   G  D  S

CTCGGTTGCA AATTCAGGTT TGGCGGCATT AATAACAGAC GGACCCGGTG GGGCAAAGCG   1560
 S  V  A   N  S  G  L   A  A  L   I  T  D   G  P  G  G   A  K  R

AATGTATGTC GGCCGGCAAA ACGCCGGTGA GACATGGCAT GACATTACCG GAAACCGTTC   1620
 M  Y  V   G  R  Q  N   A  G  E   T  W  H   D  I  T  G   N  R  S

GGAGCCGGTT GTCATCAATT CGGAAGGCTG GGGAGAGTTT CACGTAAACG GCGGGTCGGT   1680
 E  P  V   V  I  N  S   E  G  W   G  E  F   H  V  N  G   G  S  V

TTCAATTTAT GTTCAAAGAT AGAAGAGCAG AGAGGACGGA TTTCCTGAAG GAAATCCGTT   1740
 S  I  Y   V  Q  R  *

TTTTTATTTT GCCCGTCTTA TAAATTTCTT TGATTACATT TTATAATTAA TTTTAACAAA   1800

GTGTCATCAG CCCTCAGGAA GGACTTGCTG ACAGTTTGAA TCGCATAGGT AAGGCGGGGA   1860

TGAAATGGCA ACGTTATCTG ATGTAGCAAA GAAAGCAAAT GTGTCGAAAA TGACGGTATC   1920

GCGGGTGATC AATCATCCTG AGACTGTGAC GGATGAATTG AAAAAGCT                1968
```

FIG._3C

```
ANLNGTLMQY FEWYMPNDGQ HWKRLQNDSA YLAEHGITAV WIPPAYKGTS QADVGYGAYD    60
LYDLGEFHQK GTVRTKYGTK GELQSAIKSL HSRDINVYGD VVINHKGGAD ATEDVTAVEV   120
DPADRNRVIS GEHLIKAWTH FHFPGRGSTY SDFKWHWYHF DGTDWDESRK LNRIYKFQGK   180
AWDWEVSNEN GNYDYLMYAD IDYDHPDVAA EIKRWGTWYA NELQLDGFRL DAVKHIKFSF   240
LRDWVNHVRE KTGKEMFTVA EYWQNDLGAL ENYLNKTNFN HSVFDVPLHY QFHAASTQGG   300
GYDMRKLLNG TVVSKHPLKS VIFVDNHDTQ PGQSLESTVQ TWFKPLAYAF ILTRESGYPQ   360
VFYGDMYGTK GDSQREIPAL KHKIEPILKA RKQYAYGAQH DYFDHHDIVG WTREGDSSVA   420
NSGLAALITD GPGGAKRMYV GRQNAGETWH DITGNRSEPV VINSEGWGEF HVNGGSVSIY   480
VQR
```

FIG._4

Am-Lich = *B.licheniformis* Am-Amylo = *B.amyloliquefaciens* Am-Stero = *B.stearothermophilus.*

```
                                                           1              19
           1                                                              60
Am-Lich    ......MKQQ KRLYARLLTL LFALIFLLPH ......SAAA AANLNGTLMQ YFEWYMPNDG
Am-Amylo   MRGRGNMIQK RKRTVSFRLV LMCTLLFVSL ......PITK TSAVNGTLMQ YFEWYTPNDG
Am-Stearo  ......VLTF HRIIRKGWMF LLAFLLTASL FCPTGRHAKA AAPFNGTMMQ YFEWYLPDDG

                                                                          79
           61                                                            120
Am-Lich    QHWKRLQNDS AYLAEHGITA VWIPPAYKGT SQADVGYGAY DLYDLGEFHQ KGTVRTKYGT
Am-Amylo   QHWKRLQNDA EHLSDIGITA VWIPPAYKGL SQSDNGYGPY DLYDLGEFQQ KGTVRTKYGT
Am-Stearo  TLWTKVANEA NNLSSLGITA LSLPPAYKGT SRSDVGYGVY DLYDLGEFNQ KGTVRTKYGT

                                                                         139
           121                                                           180
Am-Lich    KGELQSAIKS LHSRDINVYG DVVINHKGGA DATEDVTAVE VDPADRNRVI SGEHLIKAWT
Am-Amylo   KSELQDAIGS LHSRNVQVYG DVVLNHKAGA DATEDVTAVE VNPANRNQET SEEYQIKAWT
Am-Stearo  KAQYLQAIQA AHAAGMQVYA DVVFDHKGGA DGTEWVDAVE VNPSDRNQEI SGTYQIQAWT

                                                                         197
           181                                                           240
Am-Lich    HFHFPGRGST YSDFKWHWYH FDGTDWDESR KLNRIYKF.. QGKAWDWEVS NENGNYDYLM
Am-Amylo   DFRFPGRGNT YSDFKWHWYH FDGADWDESR KISRIFKFRG EGKAWDWEVS SENGNYDYLM
Am-Stearo  KFDFPGRGNT YSSFKWRWYH FDGVDWDESR KLSRIYKFRG IGKAWDWEVD TENGNYDYLM

                                                                         257
           241                                                           300
Am-Lich    YADIDYDHPD VAAEIKRWGT WYANELQLDG FRLDAVKHIK FSFLRDWVNH VREKTGKEMF
Am-Amylo   YADVDYDHPD VVAETKKWGI WYANELSLDG FRIDAAKHIK FSFLRDWVQA VRQATGKEMF
Am-Stearo  YADLDMDHPE VVTELKNWGK WYVNTTNIDG FRLDGLKHIK FSFFPDWLSY VRSQTGKPLF
```

FIG._5A

Am-Lich = *B.licheniformis* Am-Amylo = *B.amyloliquefaciens* Am-Stero = *B.stearothermophilus.*

```
                                                                  317
         301                                                      360
Am-Lich  TVAEYWQNDL GALENYLNKT NFNHSVFDVP LHYQFHAAST QGGGYDMRKL LNGTVVSKHP
Am-Amylo TVAEYWQNNA GKLENYLNKT SFNQSVFDVP LHFNLQAASS QGGGYDMRRL LDGTVVSRHP
Am-Stearo TVGEYWSYDI NKLHNYITKT NGTMSLFDAP LHNKFYTASK SGGAFDMRTL MTNTLMKDQP

                                                                  377
         361                                                      420
Am-Lich  LKSVTFVDNH DTQPGQSLES TVQTWFKPLA YAFILTRESG YPQVFYGDMY GTKGDSQREI
Am-Amylo EKAVTFVENH DTQPGQSLES TVQTWFKPLA YAFILTRESG YPQVFYGDMY GTKGTSPKEI
Am-Stearo TLAVTFVDNH DTNPAKR.CS HGRPWFKPLA YAFILTRQEG YPCVFYGDYY GI...PQYNI

                                                                  437
         421                                                      480
Am-Lich  PALKHKIEPI LKARKQYAYG AQHDYFDHHD IVGWTREGDS SVANSGLAAL ITDGPGGAKR
Am-Amylo PSLKDNIEPI LKARKEYAYG PQHDYIDHPD VIGWTREGDS SAAKSGLAAL ITDGPGGSKR
Am-Stearo PSLKSKIDPL LIARRDYAYG TQHDYLDHSD IIGWTREGVT EKPGSGLAAL ITDGAGRSKW

                                                       483
         481                                                      540
Am-Lich  MYVGRQNAGE TWHDITGNRS EPVVINSEGW GEFHVNGGSV SIYVQR.... ..........
Am-Amylo MYAGLKNAGE TWYDITGNRS DTVKIGSDGW GEFHVNDGSV SIYVQK.... ..........
Am-Stearo MYVGKQHAGK VFYDLTGNRS DTVTINSDGW GEFKVNGGSV SVWVPRKTTV STIARPITTR

         541            559
Am-Lich  .......... ........
Am-Amylo .......... ........
Am-Stearo PWTGEFVRWH EPRLVAWP*
```

FIG._5B

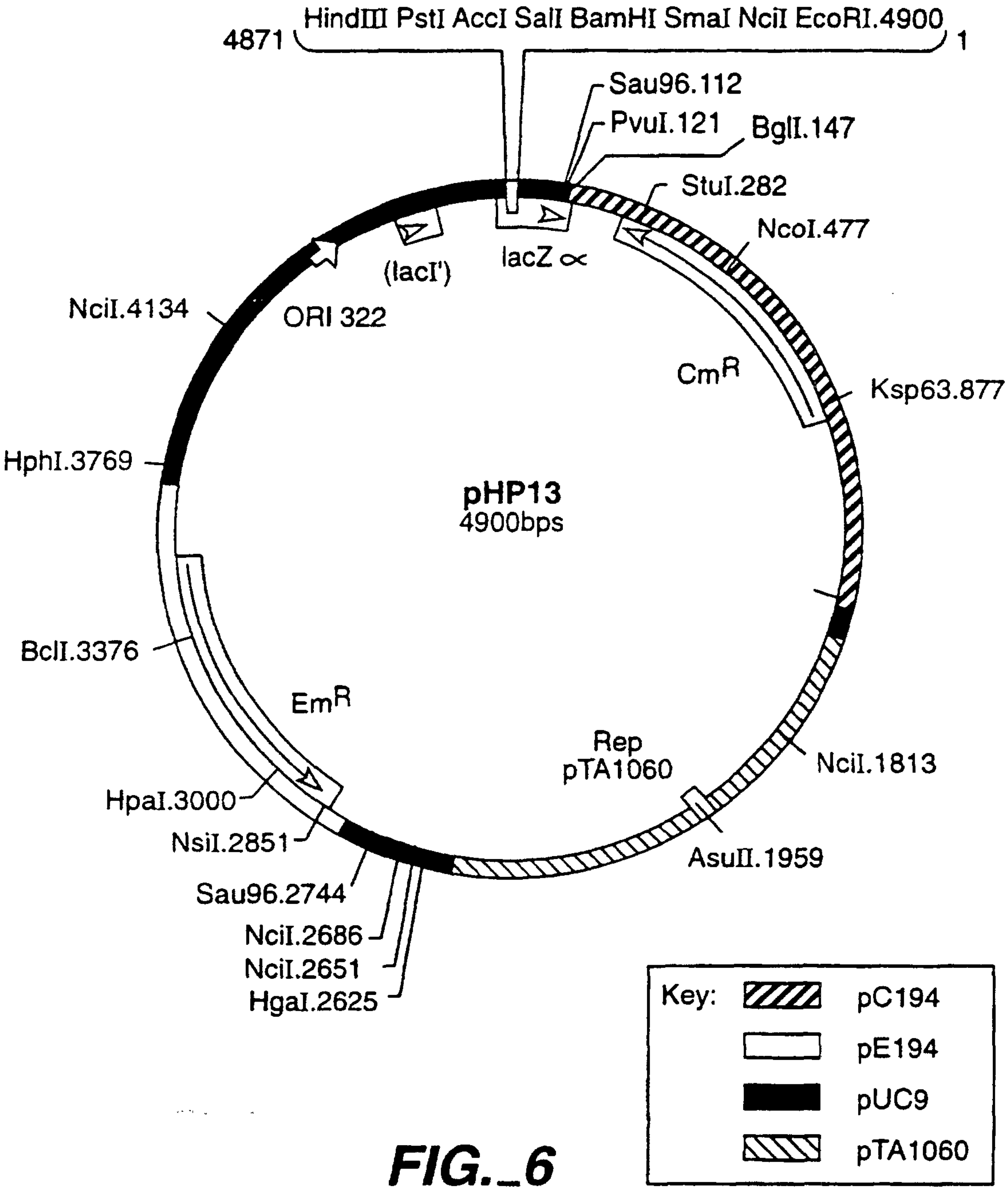

HindIII PstI AccI SalI BamHI SmaI NciI EcoRI.4900
4871
1
Sau96.112
PvuI.121
BglI.147
StuI.282
NcoI.477
Ksp63.877
NciI.1813
AsuII.1959
HgaI.2625
NciI.2651
NciI.2686
Sau96.2744
NsiI.2851
HpaI.3000
BclI.3376
HphI.3769
NciI.4134
ORI 322
(lacI')
lacZ ∝
CmR
pHP13
4900bps
EmR
Rep
pTA1060
Key:
pC194
pE194
pUC9
pTA1060


FIG._6

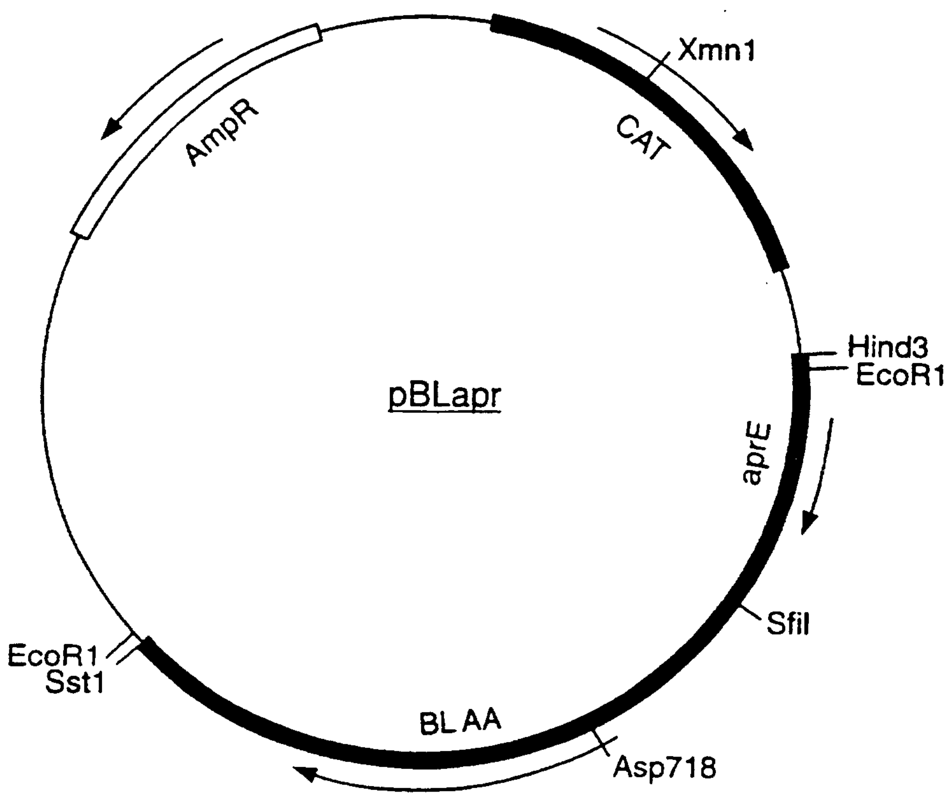
Xmn1
CAT
AmpR
Hind3
EcoR1
pBLapr
aprE
SfiI
EcoR1
Sst1
BL AA
Asp718

FIG._7

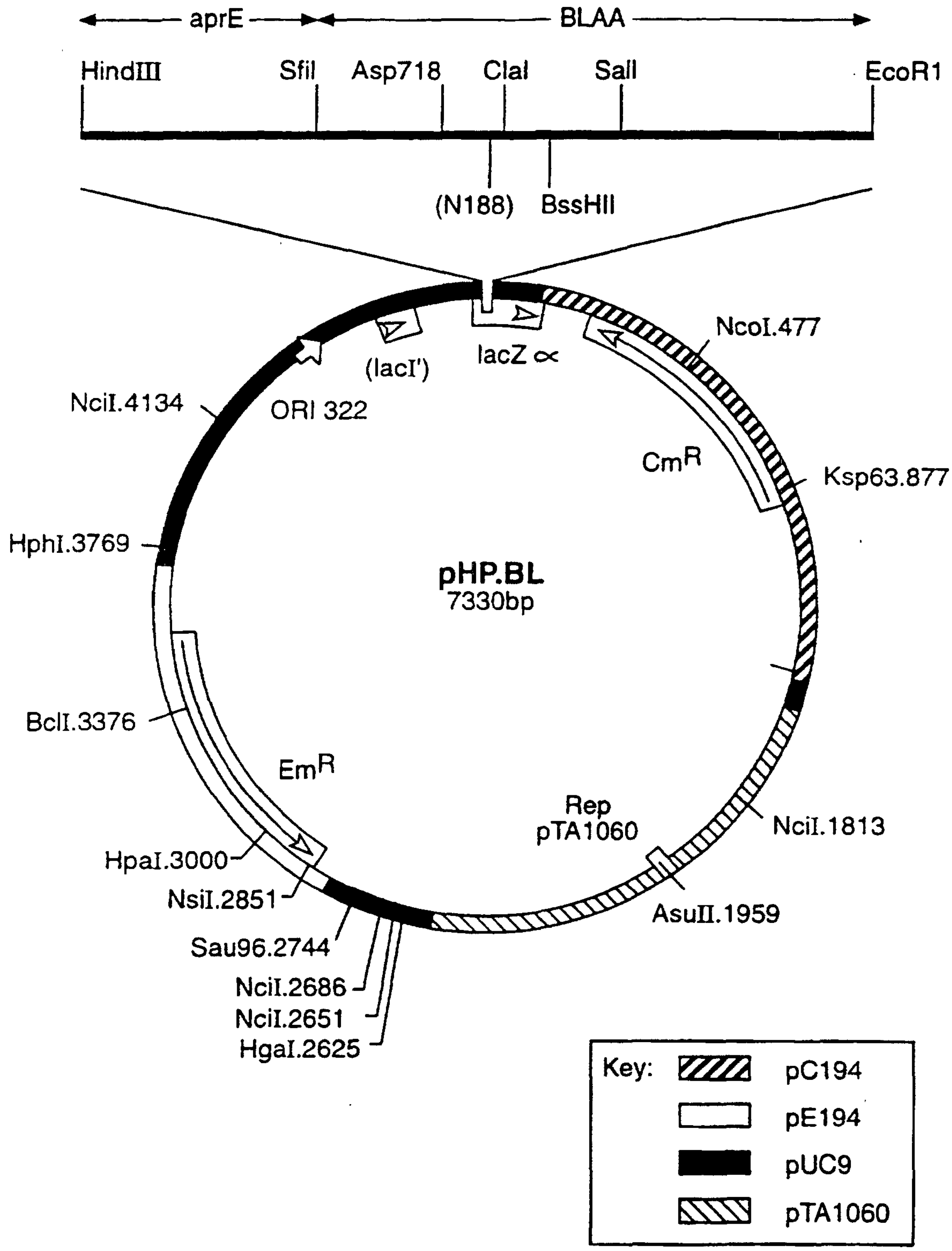


pHP.BL = pHP13 with the 2460bp HindIII-EcoRI insert from pBLapr

*FIG._8*

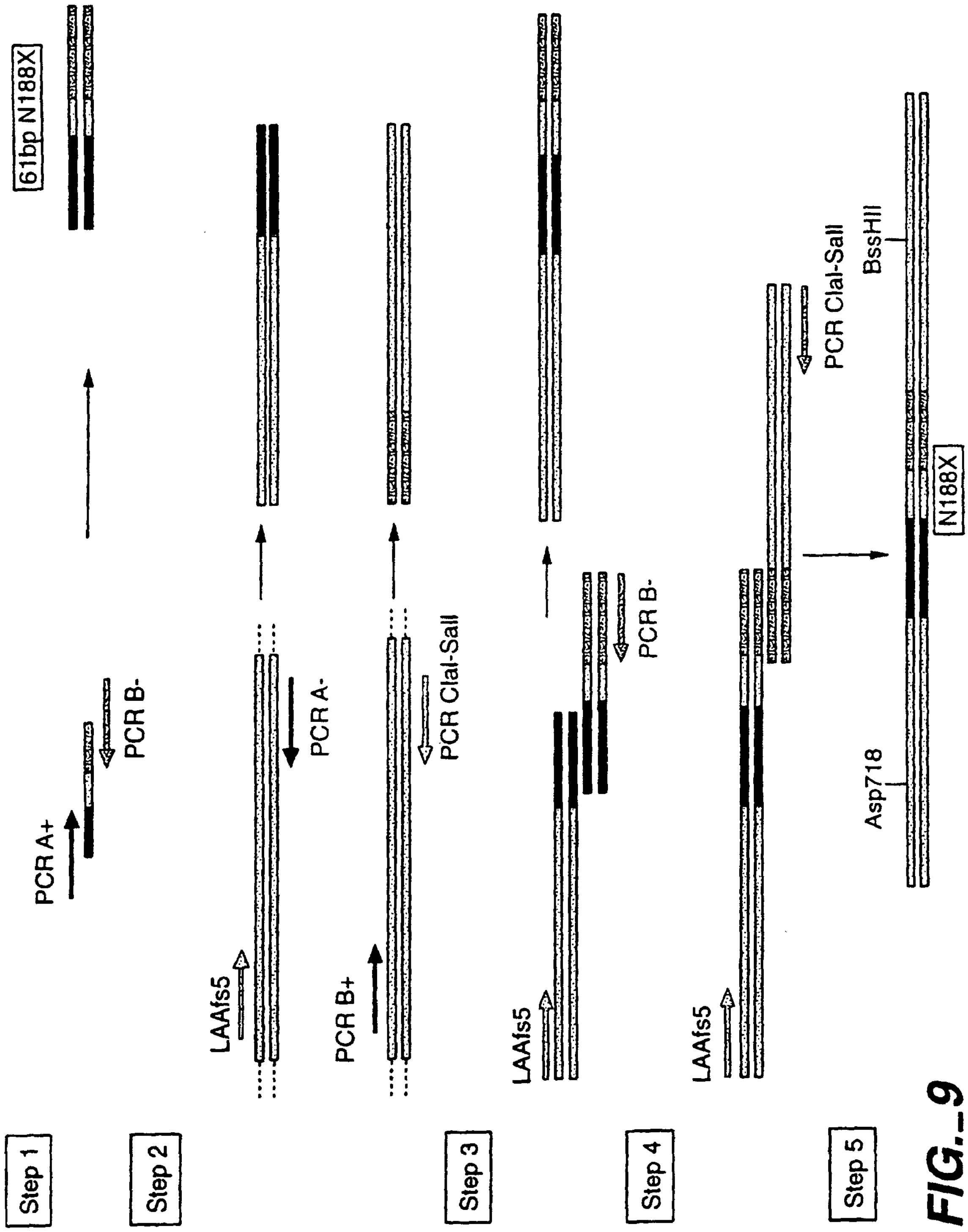
Step 1
PCR A+
PCR B-
61bp N188X
Step 2
LAAfs5
PCR A-
PCR B+
PCR ClaI-SalI
Step 3
LAAfs5
PCR B-
Step 4
LAAfs5
PCR ClaI-SalI
Step 5
Asp718
BssHII
N188X

FIG._9

SIGNAL SEQUENCE-MATURE PROTEIN JUNCTIONS IN:

*B.licheniformis* alpha-amylase.

(PstI)↓ between S A and A A A

M K Q Q K R L T A R L L T L L F A L I F L L P H S A A A A **A N L** . . . . . . . . . . . .

*B.subtilis* alkaline protease aprE.

(PstI)↓ between A and G K S

*M R S K T L W I S L L F A L T L I F T M A F S N M S A Q A* **A G K S** . . . . . . . . . . .

*B.licheniformis* alpha-amylase in pBLapr.

*M R S K T L W I S L L F A L T L I F T M A F S N M S A Q A* **A N L** . . . . . . . . . . . .

(PstI)↓ indicates the site of the restriction site in the gene

**BOLD TYPE** indicates the N-terminus of the secreted protein in *Bacillus.*

FIG._10